# EUROPEAN PATENT SPECIFICATION

(11) **EP 1 863 564 B1**
(45) Date of publication and mention of the grant of the patent: **23.11.2016**
(21) Application number: 06737480.1
(22) Date of filing: 09.03.2006
(51) Int. Cl.: A61N 1/362, A61N 1/05, A61N 1/39

(54) **COMBINED NEURAL STIMULATION AND CARDIAC RESYNCHRONIZATION THERAPY**
KOMBINIERTE NERVENSTIMULATIONS- UND HERZRESYNCHRONISATIONSTHERAPIE
THERAPIE COMBINEE DE STIMULATION NEURALE ET DE RESYNCHRONISATION CARDIAQUE

(30) Priority: 11.03.2005 US 77970; 11.03.2005 US 78460
(43) Date of publication of application: 12.12.2007
(73) Proprietor: CARDIAC PACEMAKERS, INC., St. Paul, Minnesota 55112-5798 (US)
(72) Inventor: LIBBUS, Imad, St. Paul , Minnesota 55105 (US); SPINELLI, Julio, C., Shoreview, Minnesota 55126 (US); WESTLUND, Randy, River Falls, Wisconsin 54022 (US); MOFFITT, Julia, Iowa City, Iowa 52246 (US); WANG, Sophia, H., New Brighton, Minnesota 55112 (US); KENKNIGHT, Bruce, H., Maple Grove, Minnesota 55311 (US)
(74) Representative: Pfenning, Meinig & Partner mbB
(86) International application number: PCT/US2006/008312
(87) International publication number: WO 2006/098996

(56) References cited:
- EP-A- 1 304 135
- EP-A- 1 421 973
- WO-A-99/65561
- WO-A-02/087694
- WO-A-03/020364
- WO-A-2005/113066
- WO-A1-94/00190
- US-A- 5 243 980
- US-A- 5 243 980
- US-A- 5 507 784
- US-A- 5 578 061
- US-A1- 2003 050 670
- US-A1- 2003 199 934
- US-A1- 2003 199 958
- US-A1- 2004 098 057
- US-A1- 2004 199 210
- US-B1- 6 544 270
- ZHANG Y ET AL: "Achieving regular slow rhythm during atrial fibrillation without atrioventricular nodal ablation: Selective vagal stimulation plus ventricular pacing", HEART RHYTHM, ELSEVIER, vol. 1, no. 4, 1 October 2004 (2004-10-01) , pages 469-475, XP004608379, ISSN: 1547-5271
- MURAKAWA Y ET AL: "Effect of Cervical Vagal Nerve Stimulation on Defibrillation Energy", JAPANESE HEART JOURNAL, JAPANESE HEART JOURNAL ASSOCIATION, JP, vol. 44, no. 1, 1 January 2003 (2003-01-01), pages 91-100, XP002462211, ISSN: 0021-4868

## Description

### Field of the Invention

This patent application pertains to methods and apparatus for the treatment of cardiac disease. In particular, it relates to methods and apparatus for improving cardiac function with electro-stimulatory therapy.

### Background

Following myocardial infarction (MI) or other cause of decreased cardiac output, a complex remodeling process of the ventricles occurs that involves structural, biochemical, neurohormonal, and electrophysiologic factors. Ventricular remodeling is triggered by a physiological compensatory mechanism that acts to increase cardiac output due to so-called backward failure which increases the diastolic filling pressure of the ventricles and thereby increases the so-called preload (i.e., the degree to which the ventricles are stretched by the volume of blood in the ventricles at the end of diastole). An increase in preload causes an increase in stroke volume during systole, a phenomena known as the Frank-Starling principle. When the ventricles are stretched due to the increased preload over a period of time, however, the ventricles become dilated. The enlargement of the ventricular volume causes increased ventricular wall stress at a given systolic pressure. Along with the increased pressure-volume work done by the ventricle, this acts as a stimulus for hypertrophy of the ventricular myocardium. The disadvantage of dilatation is the extra workload imposed on normal, residual myocardium and the increase in wall tension (Laplace's Law) which represent the stimulus for hypertrophy. If hypertrophy is not adequate to match increased tension, a vicious cycle ensues which causes further and progressive dilatation.

As the heart begins to dilate, afferent baroreceptor and cardiopulmonary receptor signals are sent to the vasomotor central nervous system control center, which responds with hormonal secretion and sympathetic discharge. It is the combination of hemodynamic, sympathetic nervous system and hormonal alterations (such as presence or absence of angiotensin converting enzyme (ACE) activity) that ultimately account for the deleterious alterations in cell structure involved in ventricular remodeling. The sustained stresses causing hypertrophy induce apoptosis (i.e., programmed cell death) of cardiac muscle cells and eventual wall thinning which causes further deterioration in cardiac function. Thus, although ventricular dilation and hypertrophy may at first be compensatory and increase cardiac output, the processes ultimately result in both systolic and diastolic dysfunction. It has been shown that the extent of ventricular remodeling is positively correlated with increased mortality in post-MI and heart failure patients.

Documents US2003050670 and US2003199934 disclose a device providing cardiac resynchronization therapy in heart failure patients. It includes a lead configured to be placed in the coronary sinus. EP1304135 discloses a system for providing cardiac pacing therapy, including a coronary sinus lead.

### Brief Description of the Drawings

Figs. 1A-E illustrate embodiments of the present subject matter in relation to a heart.
Fig. 2 is a system diagram of a cardiac device configured for multi-site stimulation and sensing.
Fig. 3 illustrates an embodiment of an implantable device with RF-controlled satellite units for neural stimulation.
Figs. 4A-D illustrate examples of how the intensities of ART and RCT may be individually time-varied.
FIG. 5 shows a lead adapted to deliver neural stimulation and ventricular stimulation, according to various embodiments of the present subject matter;
FIG. 6 shows a lead adapted to deliver neural stimulation and ventricular stimulation, according to various embodiments of the present subject matter;
FIG. 7 shows a lead adapted to deliver neural stimulation and ventricular stimulation, according to various embodiments of the present subject matter;
FIG. 8 shows a lead adapted to deliver neural stimulation, according to various embodiments of the present subject matter;
FIG. 9 shows a lead adapted to deliver neural stimulation, according to various embodiments of the present subject matter;
FIG. 10 shows a lead adapted to deliver neural stimulation, according to various embodiments of the present subject matter;
FIG. 11 shows a lead with a lead for transvascular stimulation, according to various embodiments of the present subject matter;
FIG. 12 shows a lead with a lead for transvascular stimulation, according to various embodiments of the present subject matter;
FIG. 13 shows a lead with at least one ring electrode adapted to deliver neural stimulation, and a tip electrode adapted to deliver ventricular stimulation, according to various embodiments of the present subject matter;
FIG. 14 shows a lead with at least one ring electrode adapted to deliver neural stimulation, and a tip electrode adapted to deliver ventricular stimulation, according to various embodiments of the present subject matter;
FIG. 15 shows a perspective view including a partial cross section of a lead, according to one embodiment of the present subject matter;
FIGS; 16A shows a device adapted to deliver neural stimulation and cardiac resynchronization therapy, according to various embodiments of the present subject matter;
FIG. 16B shows a partially hidden view of a device adapted to deliver neural stimulation and cardiac resynchronization therapy, according to various embodiments of the present subject matter;
FIG. 17A shows a device adapted to puncture vasculature, according to various embodiments of the present subject matter;
FIG. 17B shows a partially hidden view of a device to deliver neural stimulation and to puncture vasculature, according to various embodiments of the present subject matter;
FIG. 18 illustrates a block diagram of an implantable medical device, according to one embodiments of the present subject matter;
FIG. 19 shows a method for providing neural stimulation therapy and cardiac resynchronization therapy, according to one embodiment of the present subject matter;
FIG. 20 shows a system with leads placed transxiphoidally, according to one embodiment of the present subject matter;
FIG. 21A illustrates an apparatus useful for deploying one or more electrodes in a transthoracic process, according to various embodiment of the present subject matter;
FIG. 21B illustrates an apparatus useful for deploying one or more electrodes in a transthoracic process, according to various embodiment of the present subject matter;
FIG. 21C illustrates an electrode placed proximal a vessel, according to one embodiment of the present subject matter;
FIG. 22 illustrates a method for delivering cardiac resynchronization therapy in conjunction with parasympathetic response therapy for ventricular wall stress reduction, according to one embodiment of the present subject matter.

### Detailed Description

The degree to which a heart muscle fiber is stretched before it contracts is termed preload. The maximum tension and velocity of shortening of a muscle fiber increases with increasing preload. When a myocardial region contracts late relative to other regions, the contraction of those opposing regions stretches the later contracting region and increases the preload.

The degree of tension or stress on a heart muscle fiber as it contracts is termed afterload. Because pressure within the ventricles rises rapidly from a diastolic to a systolic value as blood is pumped out into the aorta and pulmonary arteries, the parts of the ventricle that first contract due to an excitatory stimulation do so against a lower afterload than do parts of the ventricle contracting later.

Overall, a myocardial region which contracts later than other regions is subjected to both an increased preload and afterload, causing uneven stress to the ventricular wall. The heart's initial physiological response to the uneven stress resulting from an increased preload and afterload is compensatory hypertrophy in those later contracting regions of the myocardium. In the later stages of remodeling, the regions can undergo atrophic changes with wall thinning due to the increased stress, and the extent of remodeling is positively correlated with mortality in heart failure patients.

One mode of remodeling is created by the ventricular conduction delays associated with heart failure and ventricular dysfunction. The condition can arise from autonomic imbalance. The automatic (or autonomic) nervous system regulates "involuntary" organs, while the contraction of voluntary (skeletal) muscles is controlled by somatic motor nerves. Examples of involuntary organs include respiratory and digestive organs, blood vessels and the heart. Often, the automatic nervous system functions in an involuntary, reflexive manner to regulate organs such as glands, muscles in the skin, the eye, the stomach, intestines and the bladder. These descriptions are not exhaustive or exclusive, but are provided for illustration.

The automatic nervous system includes, but is not limited to, the sympathetic nervous system and the parasympathetic nervous system. The sympathetic nervous system is affiliated with stress and the "fight or flight response" to patient stimulus. Among other effects, the "fight or flight response" increases blood pressure and heart rate to increase skeletal muscle blood flow, and decreases digestion to provide energy for "fighting or fleeing." The parasympathetic nervous system is affiliated with relaxation and the "rest and digest response" which, among other effects, decreases blood pressure and heart rate, and increases digestion to conserve energy. The automatic nervous system maintains normal internal function and works with the somatic nervous system.

Sympathetic and parasympathetic nerves act on the heart via beta-adrenergic and muscarinic receptors, respectively, to affect both heart rate and myocardial contractility. A predominance of sympathetic over parasympathetic stimulation of the heart, for example, increases both intrinsic heart rate (via receptors at the sino-atrial node) and the strength of ventricular contractions. Stimulation of cardiac parasympathetic nerves, on the other hand, decreases myocardial contractility and hence reduces ventricular wall stresses. Sympathetic inhibition, as well as parasympathetic activation, has also been associated with reduced arrhythmia vulnerability following a myocardial infarction, presumably by increasing collateral perfusion of the acutely ischemic myocardium and decreasing myocardial damage.

In addition to effects influencing heart rate, the automatic nervous system also impacts the health of cardiac myocytes. Increased sympathetic nervous system activity following ischemia often results in increased exposure of the myocardium to epinephrine and norepinephrine. These catecholamine examples activate intracellular pathways within the myocytes, which lead to myocardial death and fibrosis. Stimulation of the parasympathetic nerves, including the vagus nerve, inhibits this effect.

Ventricular dysynchrony and autonomic imbalance contribute to cardiac remodeling. Therapies directed at these areas are useful. For example, improving ventricular synchrony is one way to treat cardiac remodeling.

Myocardium which contracts earlier in the cycle is subjected to less stress and is less likely to undergo hypertrophic remodeling. This phenomenon is used to cause reversal of remodeling by pacing one or more sites in a heart chamber with one or more excitatory stimulation pulses during a cardiac cycle with a specified pulse output sequence. The stimulation is delivered in a manner that excites a previously stressed and remodeled region of the myocardium earlier during systole so that it experiences less afterload and preload. The pre-excitation of the remodeled region relative to other regions unloads the region from mechanical stress and allows reversal of remodeling to occur.

Multi-site pacing, in various embodiments, is applied to one chamber, but in many embodiments includes two heart chambers. For example, a chamber is paced at multiple sites with excitatory stimulation pulses in order to produce multiple waves of depolarization emanating from the pacing sites. This can produce a more coordinated contraction of the ventricle and thereby compensate for intraventricular conduction defects. Biventricular pacing is an example of a resynchronization therapy in which both ventricles are paced together. Some biventricular applications pace the ventricles with a simultaneous pulse, and others include a short delay between a right pulse and a left pulse.

Thus, patients can benefit from multi-site ventricular pacing for the purpose of improving cardiac output with more coordinated contractions and for the purpose of reducing ventricular wall stresses. Multi-site pacing also compensates effects of parasympathetic response stimulation such as reduction in cardiac output. For example, a patient's metabolism can demand higher output than is available under a parasympathetic response pacing mode, and multi-site pacing can adjust to this demand.

Clinical data has shown that cardiac resynchronization therapy (CRT), achieved through synchronized biventricular pacing, results in a significant improvement in cardiac function. It has also been reported CRT can be beneficial in preventing and/or reversing the ventricular remodeling that often occurs in post-MI and heart failure patients. As explained in detail below, the present subject matter relates to an implantable cardiac device capable of providing remodeling control therapy (RCT) by controlling ventricular activation with cardiac resynchronization pacing and providing anti-remodeling therapy (ART) by stimulating the baroreflex in order to inhibit sympathetic activity. The combined application of these two therapies provides a greater therapeutic benefit than either of them individually. The device controls ventricular activation through synchronized pacing of the right and left ventricles. In addition, the device may provide a combination of parasympathetic stimulation and sympathetic inhibition. Parasympathetic stimulation can be achieved through a nerve cuff electrode placed around the cervical vagus nerve bundle, while sympathetic inhibition can be achieved through baroreflex stimulation, either through a nerve cuff electrode placed around the aortic or carotid sinus nerve, or though a stimulation lead designed to stimulate baroreptors in the pulmonary artery. The device controls the delivery of RCT and ART independently in either an open-loop or closed-loop fashion, the latter based upon a cardiac function assessment performed by the device.

### 1. Remodeling control therapy by cardiac resynchronization pacing

Implantable cardiac devices that provide electrical stimulation to selected chambers of the heart have been developed in order to treat a number of cardiac disorders. A pacemaker, for example, is a device which paces the heart with timed pacing pulses, most commonly for the treatment of bradycardia where the ventricular rate is too slow. Atrio-ventricular conduction defects (i.e., AV block) and sick sinus syndrome represent the most common causes of bradycardia for which permanent pacing may be indicated. If functioning properly, the pacemaker makes up for the heart's inability to pace itself at an appropriate rhythm in order to meet metabolic demand by enforcing a minimum heart rate. Implantable devices may also be used to treat cardiac rhythms that are too fast, with either anti-tachycardia pacing or the delivery of electrical shocks to terminate atrial or ventricular fibrillation.

Implantable devices have also been developed that affect the manner and degree to which the heart chambers contract during a cardiac cycle in order to promote the efficient pumping of blood. The heart pumps more effectively when the chambers contract in a coordinated manner, a result normally provided by the specialized conduction pathways in both the atria and the ventricles that enable the rapid conduction of excitation (i.e., depolarization) throughout the myocardium. These pathways conduct excitatory impulses from the sino-atrial node to the atrial myocardium, to the atrio-ventricular node, and thence to the ventricular myocardium to result in a coordinated contraction of both atria and both ventricles. This both synchronizes the contractions of the muscle fibers of each chamber and synchronizes the contraction of each atrium or ventricle with the contralateral atrium or ventricle. Without the synchronization afforded by the normally functioning specialized conduction pathways, the heart's pumping efficiency is greatly diminished. Pathology of these conduction pathways and other inter-ventricular or intra-ventricular conduction deficits can be a causative factor in heart failure, which refers to a clinical syndrome in which an abnormality of cardiac function causes cardiac output to fall below a level adequate to meet the metabolic demand of peripheral tissues. In order to treat these problems, implantable cardiac devices have been developed that provide appropriately timed electrical stimulation to one or more heart chambers in an attempt to improve the coordination of atrial and/or ventricular contractions, termed cardiac resynchronization therapy (CRT). Ventricular resynchronization is useful in treating heart failure because, although not directly inotropic, resynchronization can result in a more coordinated contraction of the ventricles with improved pumping efficiency and increased cardiac output. Currently, a common form of CRT applies stimulation pulses to both ventricles, either simultaneously or separated by a specified biventricular offset interval, and after a specified atrio-ventricular delay interval with respect to the detection of an intrinsic atrial contraction or delivery of an atrial pace.

It has also been found that CRT can be beneficial in reducing the deleterious ventricular remodeling which can occur in post-MI and heart failure patients. Presumably, this occurs as a result of changes in the distribution of wall stress experienced by the ventricles during the cardiac pumping cycle when CRT is applied. The degree to which a heart muscle fiber is stretched before it contracts is termed the preload, and the maximum tension and velocity of shortening of a muscle fiber increases with increasing preload. When a myocardial region contracts late relative to other regions, the contraction of those opposing regions stretches the later contracting region and increases the preload. The degree of tension or stress on a heart muscle fiber as it contracts is termed the afterload. Because pressure within the ventricles rises rapidly from a diastolic to a systolic value as blood is pumped out into the aorta and pulmonary arteries, the part of the ventricle that first contracts due to an excitatory stimulation pulse does so against a lower afterload than does a part of the ventricle contracting later. Thus a myocardial region which contracts later than other regions is subjected to both an increased preload and afterload. This situation is created frequently by the ventricular conduction delays associated with heart failure and ventricular dysfunction due to an MI. The increased wall stress to the late-activating myocardial regions is most probably the trigger for ventricular remodeling. By pacing one or more sites in a ventricle in a manner which causes a more coordinated contraction, CRT provides pre-excitation of myocardial regions which would otherwise be activated later during systole and experience increased wall stress. The pre-excitation of the remodeled region relative to other regions unloads the region from mechanical stress and allows reversal or prevention of remodeling to occur.

### 2. Anti-remodeling therapy by modulating autonomic activity

As noted above, activity of the autonomic nervous system is at least partly responsible for the ventricular remodeling which occurs as a consequence of an MI or due to heart failure. It has been demonstrated that remodeling can be affected by pharmacological intervention with the use of, for example, ACE inhibitors and beta-blockers. Pharmacological treatment carries with it the risk of side effects, however, and it is also difficult to modulate the effects of drugs in a precise manner. The present subject matter employs electrostimulatory means to modulate autonomic activity, referred to as anti-remodeling therapy or ART. When delivered in conjunction with ventricular resynchronization pacing, such modulation of autonomic activity acts synergistically to reverse or prevent cardiac remodeling.

### 3. Device embodiments

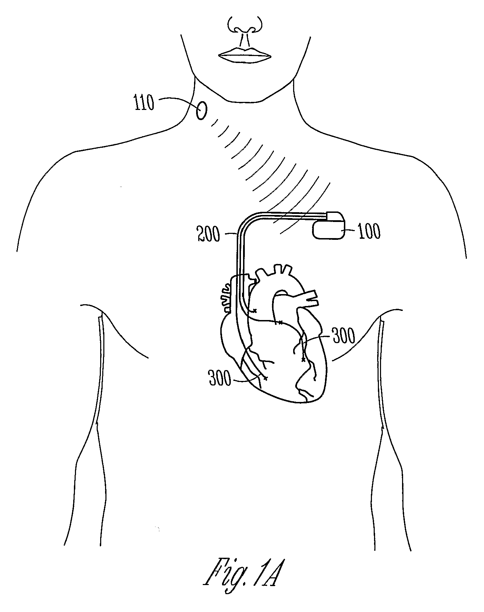

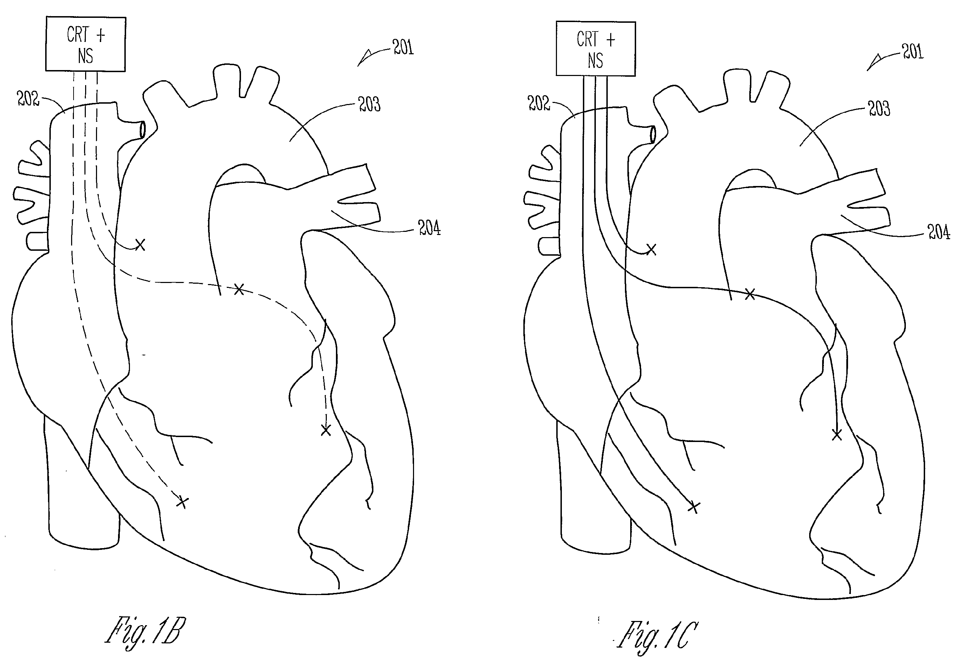

Figs. 1A-1C illustrate some device embodiments. Fig. 1A illustrates CRT performed by an implantable cardiac device and neural stimulation performed by a satellite unit. Fig. 1B illustrates an implantable medical device to perform both CRT and neural stimulation therapy using leads represented by the dotted lines and electrodes represented by "X" fed into the right atrium, right ventricle, and coronary sinus of the heart. Fig. 1C illustrates an implantable medical device with using leads represented by the dotted lines and electrodes represented by "X" epicardially positioned to perform both CRT and neural stimulation therapy. With respect to Figs. 1B and 1C, the CRT leads can be separate from the neural stimulation leads in some embodiments, and the CRT leads can be integrated with the neural stimulation leads in other embodiments. In the embodiments illustrated in Figs. 1B and 1C, a right atrium lead, a right ventricle lead and a left ventricle lead are used to perform CRT functions, and the left ventricle lead further includes a neural stimulator, such as an electrode placed in or near the coronary sinus or epicardially placed near a fat pad.

As shown in Fig. 1A, an implantable cardiac device 100 for delivering CRT is typically placed subcutaneously or submuscularly in a patient's chest with leads 200 threaded intravenously into the heart to connect the device to electrodes 300 used for sensing and pacing of the atria and/or ventricles. Electrodes may also be positioned on the epicardium by various means. A programmable electronic controller causes the pacing pulses to be output in response to lapsed time intervals and sensed electrical activity (i.e., intrinsic heart beats not as a result of a pacing pulse). The device senses intrinsic cardiac electrical activity through a sensing channel which incorporates internal electrodes disposed near the chamber to be sensed. A depolarization wave associated with an intrinsic contraction of the atria or ventricles that is detected by the device is referred to as an atrial sense or ventricular sense, respectively. In order to cause such a contraction in the absence of an intrinsic beat, a pacing pulse with energy above a certain threshold is delivered to the chamber through a pacing channel which incorporates internal electrodes disposed near the chamber to be paced. Also shown in the Fig. 1A is a satellite unit 110 which incorporates electrodes for neural stimulation and which communicates with the device 100 via a wireless link. As disclosed herein, the satellite unit 110 is capable of being positioned in a number of locations, including intravascularly and epicardially, to provide desired neural stimulation.

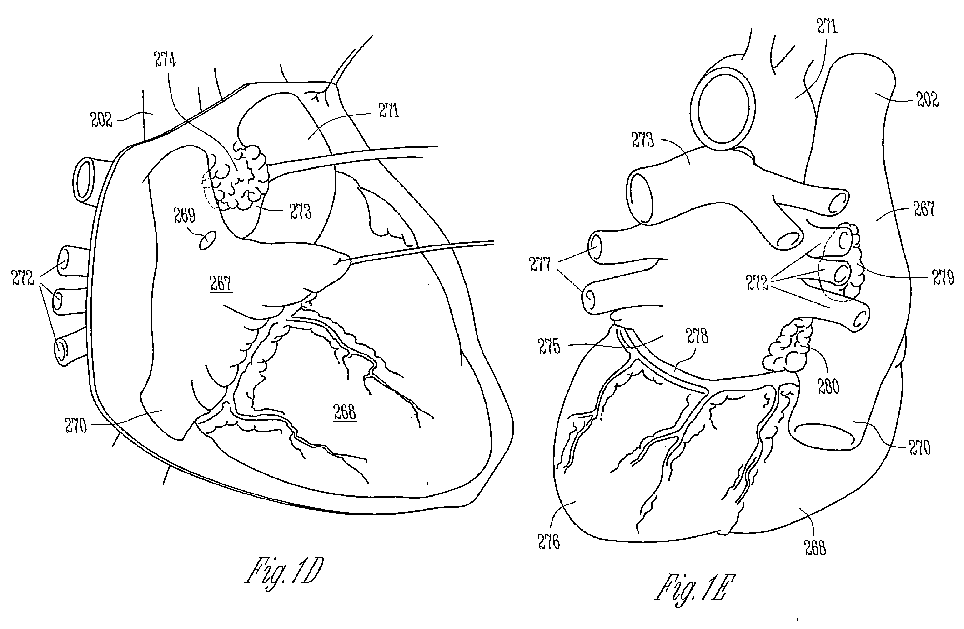

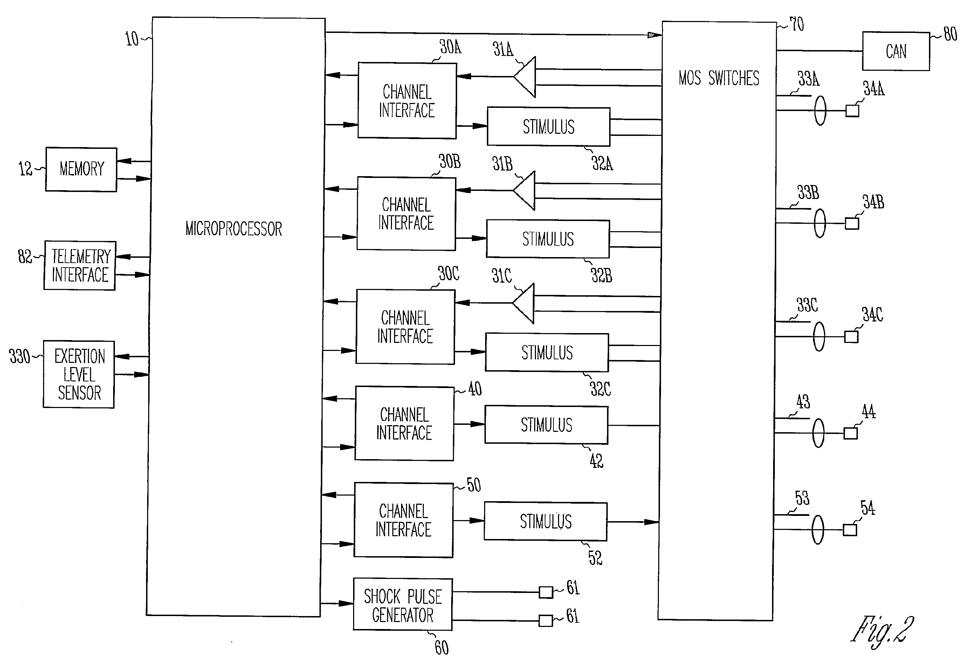

Fig. 2 shows a system diagram of an exemplary microprocessor-based cardiac device. The device is equipped with multiple sensing and pacing channels which may be physically configured to sense and/or pace multiple sites in the atria or the ventricles. The device shown in Fig. 1 can be configured for cardiac resynchronization pacing of the atria or ventricles. The multiple sensing/pacing channels may be configured, for example, with one atrial and two ventricular sensing/pacing channels for delivering biventricular resynchronization therapy, with the atrial sensing/pacing channel used to deliver the biventricular resynchronization therapy in an atrial tracking mode as well as to pace the atria if required. The controller 10 of the device is a microprocessor which communicates with a memory 12 via a bidirectional data bus. The controller could be implemented by other types of logic circuitry (e.g., discrete components or programmable logic arrays) using a state machine type of design, but a microprocessor-based system is preferable. As used herein, the term "circuitry" should be taken to refer to either discrete logic circuitry or to the programming of a microprocessor.

Figs. 1B, 1C, 1D and 1E illustrate a heart. As illustrated in Figs. 1B and 1C, the heart 201 includes a superior vena cava 202, an aortic arch 203, and a pulmonary artery 204. CRM leads pass nerve sites that can be stimulated in accordance with the present subject matter. For example, CRM leads are capable of being intravascularly inserted through a peripheral vein and into the coronary sinus, and are capable of being intravascularly inserted through a peripheral vein and through the tricuspid valve into the right ventricle of the heart (not expressly shown in the figure) similar to a cardiac pacemaker lead, and continue from the right ventricle through the pulmonary valve into the pulmonary artery. The coronary sinus and pulmonary artery are provided as examples of vasculature proximate to the heart in which a lead can be intravascularly inserted to stimulate nerves within or proximate to the vasculature. Thus, according to various aspects of the present subject matter, parasympathetic nerves are stimulated in or around vasculature located proximate to the heart by at least one electrode intravascularly inserted therein. Alternatively, a wireless stimulating device may be positioned via catheter into the vasculature located proximate to the heart. Control of stimulation and/or energy for stimulation may be supplied by another implantable or external device via ultrasonic, electromagnetic or a combination thereof.

Figs. 1D and 1E illustrate the right side and left side of the heart, respectively, and further illustrate cardiac fat pads which have nerve endings that function as baroreceptor sites. Fig. 1D illustrates the right atrium 267, right ventricle 268, sinoatrial node 269, superior vena cava 202, inferior vena cava 270, aorta 271, right pulmonary veins 272, and right pulmonary artery 273. Fig. 1D also illustrates a cardiac fat pad 274 between the superior vena cava and aorta. Nerve endings in the cardiac fat pad 274 are stimulated in some embodiments using an electrode screwed into or otherwise placed in the fat pad, and are stimulated in some embodiments using an intravenously-fed lead proximately positioned to the fat pad in a vessel such as the right pulmonary artery or superior vena cava, for example. Fig. 1E illustrates the left atrium 275, left ventricle 276, right atrium 267, right ventricle 268, superior vena cava 202, inferior vena cava 270, aorta 271, right pulmonary veins 272, left pulmonary vein 277, right pulmonary artery 273, and coronary sinus 278. Fig. 1E also illustrates a cardiac fat pad 279 located proximate to the right cardiac veins and a cardiac fat pad 280 located proximate to the inferior vena cava and left atrium. Nerve endings in the fat pad 279 are stimulated in some embodiments using an electrode screwed into the fat pad 279, and are stimulated in some embodiments using an intravenously-fed lead proximately positioned to the fat pad in a vessel such as the right pulmonary artery 273 or right pulmonary vein 272, for example. Nerve endings in the fat pad 280 are stimulated in some embodiments using an electrode screwed into the fat pad, and are stimulated in some embodiments using an intravenously-fed lead proximately positioned to the fat pad in a vessel such as the inferior vena cava 270 or coronary sinus or a lead in the left atrium 275, for example.

Shown in the Fig. 2 are three exemplary sensing and pacing channels designated "A" through "C" comprising bipolar leads with ring electrodes 34A-C and tip electrodes 33A-C, sensing amplifiers 31A-C, pulse generators 32A-C, and channel interfaces 30A-C. Each channel thus includes a pacing channel made up of the pulse generator connected to the electrode and a sensing channel made up of the sense amplifier connected to the electrode. The channel interfaces 30A-C communicate bidirectionally with microprocessor 10, and each interface may include analog-to-digital converters for digitizing sensing signal inputs from the sensing amplifiers and registers that can be written to by the microprocessor in order to output pacing pulses, change the pacing pulse amplitude, and adjust the gain and threshold values for the sensing amplifiers. The sensing circuitry of the pacemaker detects a chamber sense, either an atrial sense or ventricular sense, when an electrogram signal (i.e., a voltage sensed by an electrode representing cardiac electrical activity) generated by a particular channel exceeds a specified detection threshold. Pacing algorithms used in particular pacing modes employ such senses to trigger or inhibit pacing, and the intrinsic atrial and/or ventricular rates can be detected by measuring the time intervals between atrial and ventricular senses, respectively.

The electrodes of each bipolar lead are connected via conductors within the lead to a MOS switching network 70 controlled by the microprocessor. The switching network is used to switch the electrodes to the input of a sense amplifier in order to detect intrinsic cardiac activity and to the output of a pulse generator in order to deliver a pacing pulse. The switching network also enables the device to sense or pace either in a bipolar mode using both the ring and tip electrodes of a lead or in a unipolar mode using only one of the electrodes of the lead with the device housing or can 80 serving as a ground electrode. A shock pulse generator 60 is also interfaced to the controller for delivering a defibrillation shock via a pair of shock electrodes 61 to the atria or ventricles upon detection of a shockable tachyarrhythmia.

The controller 10 controls the overall operation of the device in accordance with programmed instructions stored in memory, including controlling the delivery of paces via the pacing channels, interpreting sense signals received from the sensing channels, and implementing timers for defining escape intervals and sensory refractory periods. The controller is capable of operating the device in a number of programmed pacing modes which define how pulses are output in response to sensed events and expiration of time intervals. Most pacemakers for treating bradycardia are programmed to operate synchronously in a so-called demand mode where sensed cardiac events occurring within a defined interval either trigger or inhibit a pacing pulse. Inhibited demand pacing modes utilize escape intervals to control pacing in accordance with sensed intrinsic activity such that a pacing pulse is delivered to a heart chamber during a cardiac cycle only after expiration of a defined escape interval during which no intrinsic beat by the chamber is detected. Escape intervals for ventricular pacing can be restarted by ventricular or atrial events, the latter allowing the pacing to track intrinsic atrial beats. CRT is most conveniently delivered in conjunction with a bradycardia pacing mode where, for example, multiple excitatory stimulation pulses are delivered to multiple sites during a cardiac cycle in order to both pace the heart in accordance with a bradycardia mode and provide pre-excitation of selected sites. An exertion level sensor 330 (e.g., an accelerometer, a minute ventilation sensor, or other sensor that measures a parameter related to metabolic demand) enables the controller to adapt the pacing rate in accordance with changes in the patient's physical activity and, as explained below, can enable the controller to modulate the delivery of neural stimulation and/or cardiac resynchronization pacing. A telemetry interface 82 is also provided which enables the controller to communicate with an external programmer or remote monitor.

Neural stimulation channels are incorporated into the device for delivering parasympathetic stimulation and/or sympathetic inhibition, where one channel includes a bipolar lead with a ring electrode 44 and a tip electrode 43, a pulse generator 42, and a channel interface 40, and the other channel includes a bipolar lead with a ring electrode 54 and a tip electrode 53, a pulse generator 52, and a channel interface 50. Other embodiments may use unipolar leads in which case the neural stimulation pulses are referenced to the can or another electrode. The pulse generator for each channel outputs a train of neural stimulation pulses which may be varied by the controller as to amplitude, frequency, and duty-cycle.

### 4. Sites for CRT and neural stimulation

In various embodiments, a neural stimulation channel uses a lead adapted to be intravascularly disposed to transvascularly stimulate an appropriate nerve, e.g., near a baroreceptor in the case of a sympathetic inhibition channel or near a parasympathetic nerve in the case of a parasympathetic stimulation channel. Some CRT devices include an atrial lead to pace and/or sense the right atrium, a right ventricle lead to pace and/or sense the right ventricle, and a left ventricle lead fed through the coronary sinus to a position to pace and/or sense the left ventricle, such as illustrated in Figs. 1B and 1C. A lead within the coronary sinus is capable of being used to transvascularly stimulate target parasympathetic nerves anatomically located on the extravascular surface of the coronary sinus at a strength sufficient to elicit depolarization of adjacent nerves, and is also capable of being used to deliver cardiac resynchronization therapy with appropriately timed pacing pulses at a site proximate to the left ventricle, for example.

According to various embodiments, the device is designed to sense a refractory period, and to deliver the neural stimulation from an electrode or electrodes within the coronary sinus during the refractory period to avoid unintentionally capturing cardiac tissue and inducing an arryhthmia such as atrial fibrillation.

Various lead embodiments implement a number of designs, including an expandable stent-like electrode with a mesh surface dimensioned to abut a wall of a predetermined blood vessel, a coiled electrode(s), a fixed screw-type electrode(s), and the like. Various embodiments place the electrode(s) inside the blood vessel, into the wall of the blood vessel, or a combination of at least one electrode inside the blood vessel and at least one electrode into the wall of the blood vessel. The neural stimulation electrode(s) can be integrated into the same lead used for CRT or in another lead in addition to CRT lead(s). In various embodiments, the neural stimulation can be performed by a satellite stimulator located intravascularly and controlled by a planet such as an implantable medical device performing CRT functions.

Transvascular leads can be used to stimulate other nerve sites. For example, an embodiment feeds a transvascular stimulation lead into the right azygos vein to stimulate the vagus nerve; and an embodiment feeds a transvascular stimulation lead into the internal jugular vein to stimulate the vagus nerve. Various embodiments use at least one lead intravascularly fed along a lead path to transvascularly apply neural stimulation and electrically stimulate a cardiac muscle, such as ventricular pacing, as part of CRT.

Other transvascular locations have been mentioned with respect to Figs. 1D and 1E. Depending on the intravascular location of the neural stimulation electrode(s), the right vagal branch, the left vagal branch or a combination of the right and left vagal branches are capable of being stimulated. The left and right vagal branches innervate different areas of the heart, and thus provide different results when stimulated. According to present knowledge, the right vagus nerve appears to innervate the right side of the heart, including the right atrium and right ventricle, and the left vagus nerve appears to innervate the left side of the heart, including the left atrium and left ventricle. Stimulation of the right vagus has more chronotropic effects because the sinus node is on the right side of the heart. Thus, various embodiments selectively stimulate the right vagus nerve and/or the left vagus nerve to selectively control contractility, excitability, and inflammatory response on the right and/or left side of the heart. Since the venous system is for the most part symmetrical, leads can be fed into an appropriate vessel to transvascularly stimulate the right or left vagus nerve. For example, a lead in the right internal jugular vein can be used to stimulate the right vagus nerve and a lead in the left internal jugular vein can be used to stimulate the left vagus nerve.

The stimulation electrode(s) are not in direct neural contact with the parasympathetic nerve when the transvascular approach to peripheral nerve stimulation is used. Thus, problems associated with neural inflammation and injury commonly associated with direct contact electrodes are reduced.

Various embodiments use at least one lead fed along a lead path and adapted to be epicardially-disposed near an appropriate neural stimulation site for vagal nerves and an appropriate site for electrically stimulating cardiac muscle, such as ventricular pacing, as part of CRT. In various embodiments, a satellite unit to provide neural stimulation is epicardially-disposed near an appropriate neural stimulation site. Such satellite units are capable of being integrated with implantable CRM devices that are performing CRT functions using either epicardial leads or transvascularly-fed leads.

According to various embodiments, the device is designed to sense a refractory period, and to deliver the neural stimulation from an electrode or electrodes within the coronary sinus during the refractory period to avoid unintentionally capturing cardiac tissue and inducing an arryhthmia such as atrial fibrillation. The myelinated vagal nerve fibers of a parasympathetic nervous system is much lower than that of myocardial tissue. Thus, when stimulating these myelinated vagal nerve fibers, parasympathetic stimulation can be applied in the absence of myocardial stimulation.

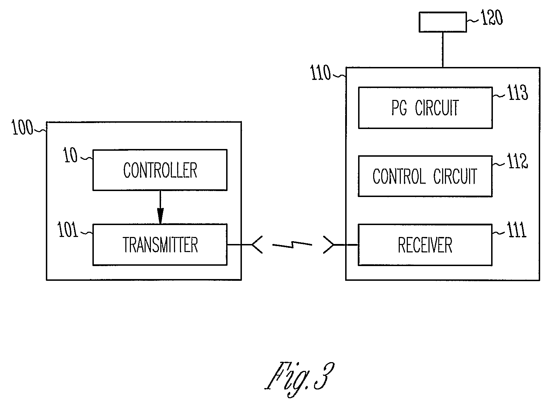

In various embodiments, the leads of the neural stimulation electrodes are replaced by wireless links, and the electrodes for providing parasympathetic stimulation and/or sympathetic inhibition are incorporated into satellite units. The wireless link may be, for example, a radio-frequency (RF) link or an acoustic link. Fig. 1A illustrates a wireless embodiment where the implantable device 100 communicates with such a satellite unit 110 via a wireless transmitter interfaced to the controller. The satellite unit 110 is an integrated assembly adapted for surgical implantation which includes a housing containing a battery and circuitry for outputting neural stimulation pulses to a target nerve (e.g., vagus, carotid sinus, or aortic nerve). Fig. 3 illustrates the functional components of the implantable device 100 and satellite unit 110 relevant to this embodiment. The implantable device includes a wireless transmitter 101 (e.g., either an RF transmitter or an acoustic transducer) interfaced to the controller 10 for transmitting commands, and the satellite unit 110 includes a wireless receiver 111 (e.g., either an RF receiver or a microphone for receiving acoustic signals) interfaced to control circuitry 112 for receiving the commands. The control circuitry 112 translates the received commands and causes pulse generation circuitry 113 to output appropriate stimulation pulses to the external electrode 120.

Increased sympathetic nervous system activity following ischemia often results in increased exposure of the myocardium to epinephrine and norepinephrine. These catecholamines activate intracellular pathways within the myocytes, which lead to myocardial death and fibrosis. Stimulation of the parasympathetic nerves (vagus) inhibits this effect. According to various embodiments, the present subject matter selectively activates the vagal cardiac nerves in addition to CRT in heart failure patients to protect the myocardium from further remodeling and arrhythmogenesis. Other potential benefits of stimulating vagal cardiac nerves in addition to CRT include reducing inflammatory response following myocardial infarction, and reducing the electrical stimulation threshold for defibrillating. For example, when a ventricular tachycardia is sensed, vagal nerve stimulation is applied, and then a defibrillation shock is applied. The vagal nerve stimulation allows the defibrillation shock to be applied at less energy.

### 5. Assessment of cardiac function

In one embodiment, the delivery of RCT and/or ART is modulated in accordance with an assessment of cardiac function performed by the implantable device. One means by which cardiac function may be assessed is by measuring cardiac output and comparing it with the patient's measured exertion level. Cardiac output may be measured by an impedance technique in which transthoracic impedance is measured and used compute stroke volume. In one embodiment, the exertion level sensor is a minute ventilation sensor which includes an exciter and an impedance measuring circuit. The exciter supplies excitation current of a specified amplitude (e.g., as a pulse waveform with constant amplitude) to excitation electrodes that are disposed in the thorax. Voltage sense electrodes are disposed in a selected region of the thorax so that the potential difference between the electrodes while excitation current is supplied is representative of the transthoracic impedance between the voltage sense electrodes. The conductive housing or can may be used as one of the voltage sense electrodes. The impedance measuring circuitry processes the voltage sense signal from the voltage sense electrodes to derive the impedance signal. Further processing of the impedance signal allows the derivation of signal representing respiratory activity and/or cardiac blood volume, depending upon the location the voltage sense electrodes in the thorax. (See, e.g., U.S. Patent Nos. 5,190,035 and 6,161,042, assigned to the assignee of the present invention.) If the impedance signal is filtered to remove the respiratory component, the result is a signal that is representative of blood volume in the heart at any point in time, thus allowing the computation of stroke volume and, when combined with heart rate, computation of cardiac output. The stroke volume integrated over time (or averaged and multiplied by heart rate) gives the patient's cardiac output. A look-up table or other function may be used to compute what cardiac output is considered adequate for a given exertion level. Measurement of cardiac output or a determination of the adequacy of the cardiac output may be used by the device to modulate the delivery of RCT and/or ART.

Another means for assessing cardiac function is by determining the autonomic balance of the patient. It is well-known that an increase in the activity of the sympathetic nervous system may be indicative of metabolic stress and the need for increased cardiac output. One means by which increased sympathetic activity may be detected is via spectral analysis of heart rate variability. Heart rate variability refers to the variability of the time intervals between successive heart beats during a sinus rhythm and is primarily due to the interaction between the sympathetic and parasympathetic arms of the autonomic nervous system. Spectral analysis of heart rate variability involves decomposing a signal representing successive beat-to-beat intervals into separate components representing the amplitude of the signal at different oscillation frequencies. It has been found that the amount of signal power in a low frequency (LF) band ranging from 0.04 to 0.15 Hz is influenced by the levels of activity of both the sympathetic and parasympathetic nervous systems, while the amount of signal power in a high frequency band (HF) ranging from 0.15 to 0.40 Hz is primarily a function of parasympathetic activity. The ratio of the signal powers, designated as the LF/HF ratio, is thus a good indicator of the state of autonomic balance, with a high LF/HF ratio indicating increased sympathetic activity. An LF/HF ratio which exceeds a specified threshold value may be taken as an indicator that cardiac function is not adequate. A cardiac rhythm management device can be programmed to determine the LF/HF ratio by analyzing data received from its atrial ventricular sensing channels. The intervals between successive atrial or ventricular senses, referred to as beat-to-beat or BB intervals, can be measured and collected for a period of time or a specified number of beats. The resulting series of RR interval values is then stored as a discrete signal and analyzed to determine its energies in the high and low frequency bands as described above. Techniques for estimating the LF/HF ratio based upon interval data are described in commonly assigned U.S. Patent Application Ser. Nos. 10/436,876 filed May 12, 2003, and 10/669,170 filed September 23, 2003.

Other means of assessing cardiac function may also be employed to modulate the delivery of ART and/or RCT. The impedance technique for measuring cardiac output discussed above may also be used to measure ventricular volumes at various stages of the cardiac cycle such as end-diastolic and end-systolic volumes and used to compute parameters reflective of cardiac function such as ejection fraction. The implantable device may also be equipped with other sensing modalities such as a pressure transducer. Such a pressure transducer may be attached to an intravascular lead and be appropriately disposed for measuring diastolic filling pressures and/or systolic pulse pressures.

### 6. Implementation examples

In an embodiment, an implantable device for delivering cardiac therapy to post-MI patients includes one or more pacing channels for delivering pacing pulses to one or more ventricular sites and a sympathetic inhibition channel for stimulating nerves which inhibit sympathetic nerve activity. The controller is programmed to deliver remodeling control therapy (RCT) by delivering ventricular pacing in a cardiac resynchronization mode which pre-excites a region of the ventricular myocardium so as to mechanically unload that region during systole. The cardiac resynchronization therapy may be delivered as biventricular pacing where one of the ventricles is pre-excited relative to the other as determined by a programmed biventricular offset interval. Alternatively, in patients suffering from delayed activation of the left ventricle, a left ventricle-only resynchronization pacing mode may be employed. In another embodiment, the pacing therapy may be delivered as multi-site ventricular pacing where at least one of the ventricles is paced at a plurality of sites so as to pre-excite one or more of the sites relative to the other sites. In any case, the ventricular pacing may be delivered in a non-atrial tracking mode where a ventricular escape interval is defined between ventricular paces, or in an atrial tracking mode where the ventricular paces are delivered after a defined atrio-ventricular escape interval following an atrial sense. In a patient who is chronotropically incompetent, an atrial pacing channel may also be provided for pacing the atria, with the ventricular pace(s) delivered upon expiration of the atrio-ventricular escape interval following the atrial pace.

The controller is further programmed to delivery anti-remodeling therapy (ART) in conjunction with the RCT by inhibiting sympathetic nerve activity via the sympathetic inhibition channel. The sympathetic inhibition channel may include a pulse generator for outputting neural stimulation pulses and a lead incorporating an electrode adapted for disposition near an arterial baroreceptor or afferent nerve of a baroreflex arc. Stimulation of the baroreflex arc then results in inhibition of sympathetic activity. The electrode of the sympathetic inhibition channel may be intravascularly positioned in a blood vessel or elsewhere proximate to a baroreceptor or afferent nerve such as in a pulmonary artery or a cardiac fat pad. In another embodiment, the device may further include a parasympathetic stimulation channel, where the anti-remodeling therapy delivered by the controller further includes stimulation of parasympathetic nerve activity, and the parasympathetic stimulation channel includes a pulse generator for outputting neural stimulation pulses and an electrode for stimulating a parasympathetic nerve. The electrode may be a nerve cuff electrode adapted for disposition around a parasympathetic nerve or an intravascular electrode for transvascularly stimulating a parasympathetic nerve adjacent to a blood vessel. As described above, for either the sympathetic inhibition channel or the parasympathetic stimulation channel, the electrode and pulse generator may also be incorporated into a satellite unit which includes an RF receiver. The implantable device then further comprises an RF transmitter interfaced to the controller for controlling the operation of the satellite unit via an RF link.

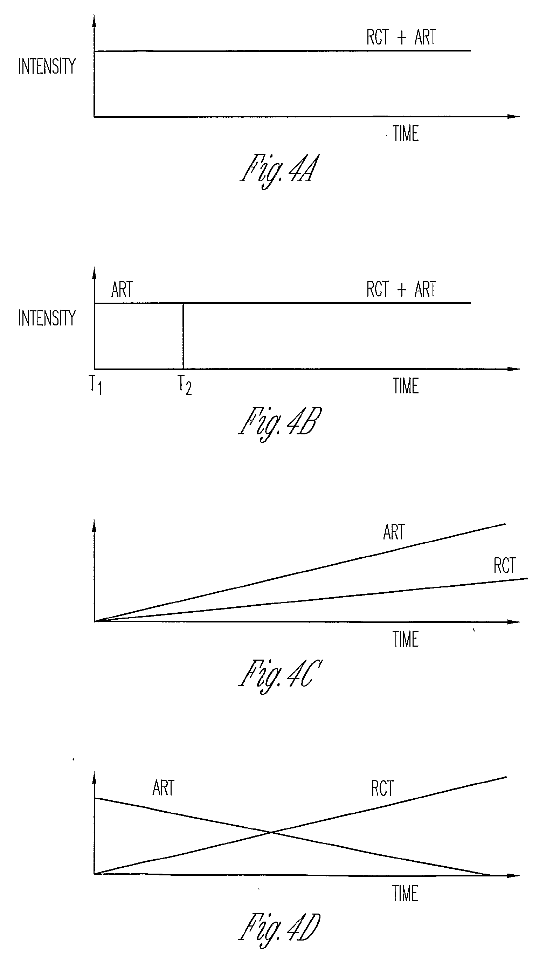

The device may be programmed to deliver RCT and ART in open-loop fashion where the RCT and ART are delivered simultaneously or separately at programmed intervals. The RCT and ART may be delivered at a constant or time-varying intensity, where the intensity of the ART may be adjusted by varying the amplitude, frequency, or duty-cycle of the neural stimulation pulses, and the intensity of the RCT may be adjusted by varying one or more parameters which affect the amount of pre-excitation delivered to the ventricles such as the biventricular offset interval and the atrio-ventricular escape interval. Figs. 4A through 4D illustrate examples of how the level of RCT and ART may be varied with respect to time. In Fig. 4A, both RCT and ART are delivered simultaneously with each type of therapy maintained at a constant intensity. In Fig. 4B, both RCT and ART are delivered at a constant intensity, but ART starts at a time T₁ while RCT starts at a time T₂. In Figs. 4C and 4D, the intensity of each type of therapy is modulated with respect to time. Fig. 4C shows the intensity of both the RCT and ART increasing with respect to time, while Fig. 4D shows the intensity of the RCT increasing while the intensity of the ART decreases. The relative intensities of sympathetic inhibition and parasympathetic stimulation may also be separately modulated.

In another embodiment, the device is programmed to deliver RCT and ART in closed-loop fashion, where the intensities of RCT and ART are modulated in accordance with an assessment of cardiac function performed by the controller. The device may also separately modulate the intensities of parasympathetic stimulation and sympathetic inhibition which are delivered as part of the ART in accordance with the assessment of cardiac function. Cardiac function may be assessed by the device using several different modalities, either alone or in combination. In one embodiment, the device incorporates a sensor for measuring cardiac output, and the controller is programmed to modulate the delivery of RCT and ART in accordance with the measured cardiac output. As described above, such a cardiac output sensor may be a trans-throracic impedance measuring circuit. Another means for assessing cardiac function is an arterial blood pressure sensor, where the controller is programmed to modulate the delivery of RCT and ART in accordance with the measured blood pressure. The blood pressure sensor may take the form of a pressure transducer and lead adapted for disposition within an artery. Alternatively, a measure of the patient's respiratory activity taken by a minute ventilation sensor may be used as a surrogate for blood pressure. Cardiac function may also be assessed by measuring the patient's exertion level (e.g., using either a minute ventilation sensor or an accelerometer) together with a measure of cardiac output and/or blood pressure, where the controller is then programmed to modulate the delivery of RCT and ART in accordance with the combined measurements.

In another embodiment, the cardiac function assessment includes an assessment of the patient's autonomic balance. Autonomic balance may be assessed directly with a sensing channel for measuring electrical activity in sympathetic and parasympathetic nerves with appropriately positioned sensing electrodes, or if the patient is chronotropically competent, by measuring the intrinsic heart rate. As described above, measuring heart rate variability provides one means for assessing autonomic balance. Thus, the device may include circuitry for measuring and collecting time intervals between successive intrinsic beats, referred to as a BB interval, where the BB interval may be an interval between successive atrial or ventricular senses. The device stores the collected intervals as a discrete BB interval signal, filters the BB interval signal into defined high and low frequency bands, and determines the signal power of the BB interval signal in each of the low and high frequency bands, referred to LF and HF, respectively. The device then computes an LF/HF ratio and assesses autonomic balance by comparing the LF/HF ratio to a specified threshold value.

In certain embodiments, data gathered by the device in performing the assessment of cardiac function performed is transmitted to a remote monitor via an RF telemetry link. The remote monitor may record the data for later analysis by a clinician and/or transmit it to another location over a network such as the internet. In response to network communications, the remote monitor may also program the implantable device via the RF telemetry link in order to modify the delivery of RCT and ART.

### 6. Lead Embodiments for applying CRT and NS therapy

The present subject matter relates to devices and methods to reduce ventricular wall stress and provides a therapy that combining cardiac resynchronization therapy and neural stimulation to induce parasympathetic response. Various aspects of the present subject matter include application of the combined therapy enabled by a single, integrated lead for placement in the left side of the heart capable of stimulating nerve fibers to induce parasympathetic response. The lead is capable of being used to provide cardiac resynchronization therapy, such as when combined with a lead adapted for placement in the right side of the heart for stimulation of the right ventricle. Alternative embodiments position the lead elsewhere.

What follows is a description of an implantable cardiac device used to practice various forms of therapy. An implantable cardiac device is typically placed subcutaneously or submuscularly in a patient's chest with leads running intravenously into the heart to connect the device to electrodes used for sensing and stimulation. Leads can also be positioned on the epicardium by various means. A programmable electronic controller causes the stimulus pulses to be output in response to lapsed time intervals and sensed electrical activity (i.e., intrinsic heart beats not as a result of a stimulus pulse). The device senses intrinsic cardiac electrical activity by means of electrodes disposed near the chamber to be sensed. A depolarization wave associated with an intrinsic contraction of the atria or ventricles that is detected by the device is referred to as an atrial sense or ventricular sense, respectively. In order to cause such a contraction in the absence of an intrinsic beat, a stimulus pulse (also referred to as a pace or pacing pulse when delivered in order to enforce a certain rhythm) with energy above a certain threshold is delivered to the chamber.

In various embodiments, an electrical stimulus originates from a central device and travels over conductive channels to arrive at an intended location. For example, multiple embodiments include a cardiac rhythm device and one or more leads running from the device to the heart. In one embodiment, the device includes a housing used to sense patient information, and in some cases the housing is used to deliver electrical stimulation. Additionally, various embodiments use electrodes present on the one or more leads to sense and deliver electrical signals.

Thus, in various embodiments, the device uses information gathered from sensors to deduce cardiac function. Various embodiments include a combination of cardiac resynchronization therapy and neural stimulation applied in a closed-loop manner in response to physiological parameters, such as heart rate changes induced by stimulation from the device, or intrinsic heart rate changes.

For example, selective stimulation of autonomic epicardial ganglia within cardiac fat pads, the fat pad located proximal the inferior vena cava-left atrial junction ("IVC-LA"), activates the parasympathetic nervous system to produce some of the benefits mentioned here. In various embodiments, this stimulation is delivered by an implantable cardiac device via an electrode incorporated into a lead. In various embodiments, the electrode is incorporated within a lead used for cardiac rhythm management therapy to the heart. A pulse generator delivers electrical stimulation via an electrode and stimulates the parasympathetic nerves that extend proximal to the electrode. Electrical stimulation of the parasympathetic nervous system can be, for example, in the form of a square-wave or truncated exponential pulse train at a frequency of between 5 and 50 Hz. The result of such electrical stimulation is a slowing of sinus rhythm due to increased parasympathetic activity acting on the sino-atrial node as well as a negative inotropic effect which decreases ventricular wall stresses during systole.

Examples of these systems include devices programmed to modulate the delivery of parasympathetic stimulation in accordance with a sensed parameter which reflects the patient's demand for cardiac output and the patient's actual cardiac output. In some embodiments, the device measures the patient's exertion level with one or both of a minute ventilation sensor and an accelerometer and delivers parasympathetic stimulation only when the measured exertion level is below a specified limit value.

In additional examples, the extent of parasympathetic stimulation varies inversely with measured exertion. For example, in one embodiment, the device measures the patient's cardiac output and delivers parasympathetic stimulation either in proportion to the measured cardiac output or only when the cardiac output exceeds a specified limit value. In another embodiment, measurements of cardiac output and exertion level are combined to provide a composite parameter that indicates the adequacy of the measured cardiac output. In various embodiments, a look-up table is used to match a particular exertion level with a minimum cardiac output considered to be adequate. The device is programmed to deliver parasympathetic stimulation only if cardiac output is at a level considered to be adequate to meet metabolic demand.

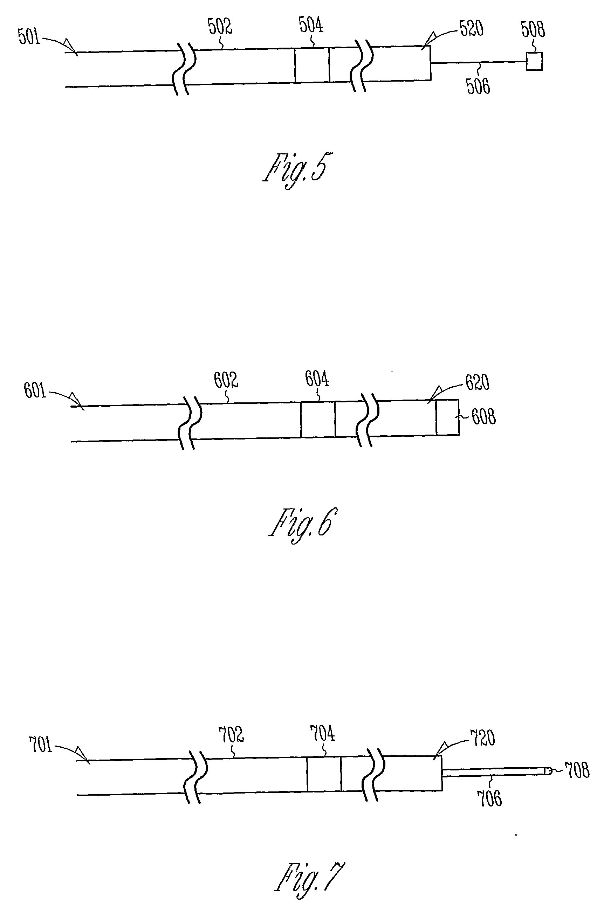

FIGS. 5-10 illustrate various embodiments of leads and lead assemblies adapted to deliver neural stimulation and ventricular stimulation. Leads illustrated include at least one electrode capable of delivering an electrical pulse to a patient, and various embodiments include sensors capable of measuring physiological parameters of a patient. Some embodiments can employ, for example, micro-electrical mechanical systems (MEMS) technology to gather physiological data. It should be noted that these illustrations are not drawn to scale, and should not be read as limiting other aspects and embodiments of the present subject matter.

According to various examples used for providing cardiac resynchronization therapy, at least one electrode is placed to stimulate the left ventricle. In various embodiments, the lead for cardiac resynchronization therapy is placed within the coronary sinus, an area which is proximal to parasympathetic ganglia. Parasympathetic ganglia reside in epicardial fat pads, including the fat pad bounded by the left atrium and the inferior vena cava.

The leads illustrated in FIGS. 5-10 include electrodes adapted for stimulation of the ganglia in the fat pad that is proximal to the coronary sinus. In particular, FIGS. 5-7 include transvascular electrodes for stimulation through the wall of the coronary sinus, through various pathways to a vagal nerve proximal the fat pad bounded by the inferior vena cava and the left atrium (IVC-LA fat pad).

various embodiments of the present subject matter deliver neural stimulation in addition to the cardiac resynchronization therapy in a ventricle, using a common lead. The following examples include embodiments within this scope. These lead illustrations should not be read as limiting other aspects and embodiments of the present subject matter.

FIG. 5 illustrates one embodiment of a lead assembly for implantation of one or more electrodes into a coronary vein on the left side of the heart according to the present subject matter. The lead, in various embodiments, includes a main lead body 102 made of an electrically insulative material. In one embodiment, the lead assembly is shaped like a flexible cylinder. The main lead body 502 includes a proximal portion 501 and a distal portion 520. In various embodiments, one or more lumen extend through the main lead body from approximately the proximal portion 501 to approximately the distal portion 520. In one embodiment, a main lead body lumen extends from a proximal portion 501 to the distal portion and terminates in an opening in a distal end.

In various embodiments, the main lead body 502 is constructed of a polymer surrounding a reinforcement material. Various embodiments include a biocompatible polymer, such as silicone rubber, but other materials are within the scope of the present subject matter. In one embodiment, the reinforcement material is a coiled wire, giving the flexible body rigidity in axial compression, but flexibility when stressed approximately orthogonal to an axis defining the center-line of the lead body. In an embodiment, the coiled wire is used as a conductor in addition to its use as reinforcement material. In various embodiments, the lumen passes through the coiled wire. In some embodiments, the coiled wire includes predetermined bends, resulting in a mechanical bias. For example, in one embodiment, the coiled wire includes a set of bends adapted to assist placement of the main lead body 502 in a coronary sinus, and in some embodiments, other cardiac veins. In some lead embodiments, the insulator itself includes a set of bends adapted to assist placement of the main lead body. In some examples, reinforcement material placed in the distal portion 520 has a bias resulting in a curve such that the distal portion bends in the absence of a guidewire and straightens when a guidewire is placed in the lumen running through the main lead body 502.

According to various embodiments, the main lead body 502 includes a first electrode 504 adapted to stimulate nerves that innervate the heart. Various electrode embodiments include at least one conductive band that at least partially circumscribes the main lead body 502. The first electrode is integrated into the main lead body 502 in some embodiments. For example, some embodiments use an exposed portion of the coil that is used to add axial stiffness to the main lead body 502 as the electrode. Other electrode designs can be used.

In various embodiments, the lead also includes a second lead body slidably disposed in a lumen of the main lead body. A second lead body such as the stimulation element 506 is slidably placed through a lumen of the main lead body 502. In various embodiments, the stimulation element runs from approximately the proximal portion 501 to approximately the distal portion 520, and in one embodiment, from approximately the proximal portion to an opening in the distal end, and through the opening. In one embodiment, the stimulation element is positioned after establishing capture of the heart, and is then fixed in position.

In various embodiments, the stimulation element 506 is a substantially flexible construction adapted for passage through patient vasculature. For example, some embodiments of the stimulation element 506 include a metallic core surrounded by an insulative material. In one example, the stimulation element 506 includes a biocompatible metallic structure including a titanium alloy, the structure being at least partially insulated, e.g. coated or covered, in a polymer. In additional examples, the flexible construction demonstrates a varied number of gradations in cross-sectional area resulting in a gradually varying diameter.

Some examples of the present subject matter stimulate the left ventricle with an electrode placed in a cardiac vein. In various embodiments, the illustrated device includes a second electrode 508 which is adapted for placement proximal the left ventricle, and which is adapted for stimulation of the left ventricle. In some embodiments, the electrode comprises a portion of the stimulation element 506, and in one embodiment, the stimulation element is an insulated wire, with an exposed portion comprising the second electrode 508.

It should be noted that various designs intended to electrically isolate the first electrode 504 from the second electrode 508 are within the scope of the present subject matter. For example, in one embodiment, the first electrode is constructed from a portion of coiled wire, and this coiled wire is electrically insulated in the main lead body 502 both from other lumen(s) and from areas external to the main lead body 502. Additional embodiments of the present subject matter offer added advantages and features. For example, the wall of the lumen is coated with a lubricious coating or a polymer with a low coefficient of friction to reduce friction between a guide wire and the wall of the lumen. Additional lumens can be provided, with some including an additional channel for a guide wire or other apparatus.

Leads made in conformance with the present subject matter are inserted in a number of different ways. For example, a guide catheter is inserted and then the lead passed through the guide catheter until it is properly positioned. The lead is coated with a lubricious coating to reduce friction in the guide catheter. The guide catheter can then be retracted. Additionally, a guide wire is advanced to the implant site alone or through a guide catheter.

Using a lumen, a lead is slid over the guide wire until the lead is properly positioned. For example, the lead is slid until a first electrode is placed in a desired position to capture vagal nerves during stimulation. The guide wire or guide catheters can then be retracted. In embodiments using a stimulation element 506, the stimulation element 506 can take the place of the guide wire, and is left in position for use as a stimulation device for the left ventricle.

Also, a lead is temporarily fixed to a guide catheter. The fixate is designed to dissolve in body fluids. The lead can then be inserted along with the guide catheter. After the electrode is in place and the fixate dissolves, the guide catheter is retracted. It should be noted that modem techniques, including modem angiography techniques, fall within the scope of the present subject.

FIG. 6 illustrates one embodiment of a lead for implantation of an electrode into a coronary vein on the left side of the heart. In various embodiments, the lead includes a main lead body 602, with a proximal portion 601, and a distal portion 620. The lead also includes a first electrode 604 and a second electrode 608. The first electrode 604, in various embodiments, is integrated into the main lead body 602, and in additional embodiments, includes features for transvascular stimulation of parasympathetic ganglia located in the IVC-LA fat pad. In one example, the first electrode 604 is a conductive band at least partially circumscribing the main lead body 602.

The second electrode 608, in various embodiments, is also integrated into the main lead body 602. In various embodiments, the second electrode 608 includes features adapted to stimulate the left ventricle. In some examples, the second electrode 608 includes a tip having features compatible with tissue growth. For example, in one embodiment the second electrode includes a porous screen which is adapted to foster connection through fibrous growth of myocardial tissue into the pores. In one embodiment the porous screen is made from platinum iridium, however, other materials include diamond, iridium oxide, titanium nitride, titanium oxide, platinum, titanium, and tantalum.

In various embodiments, the first electrode and the second electrode are electrically isolated from each other. In some examples, the first integrated electrode 604 comprises an exposed portion of a conductive coil running the length of the main lead body 602. In these examples, the second electrode is connected either with a secondary coaxial coil, or with a conductor. It should be noted that various embodiments, however, include multiple conductors running through portion of the main lead body 602 which provide electrical communication paths between the electrodes and the proximal portion 601. In one embodiment, a pair of cables extend through portions of the main lead body 602 and provide circuits capable of connecting the first electrode and the second electrode to terminals located at the proximal portion of the main lead body 602. In an additional embodiment, cables extend through portions of a lumen located in the main lead body 602. In one embodiment, the lumen for conductor passage is coated with a lubricant, including biocompatible electrically resistant lubricants.

In various embodiments, the main lead body 602 includes a lumen for a wire, such as a stylet, a guidewire, or a push-wire. In various embodiments, the guidewire is adapted to add rigidity to the main lead body 602 when bending along an axis orthogonal to a centerline coaxial to the length of the main lead body. However, various guidewires are used, adding various levels of rigidity. Additionally, one or more coils disposed in the main lead body can include a mechanical bias, and the slidable placement of the guidewire within a lumen can temporarily remove the mechanical bias for implantation steps. Upon successful implantation, the guidewire is removed, restoring a mechanical bias. In various embodiments, a mechanical bias is useful for adapting the lead to the patient's physiology, including moving one or more conductors into improved electrical communication with aspects of patient physiology.

It should also be noted that the distal end 620 of the main lead body 602 can feature an opening for lumen termination. In these examples, the lead is implanted using over-the-wire methodology, rather than stylet methodology. However, each embodiment is within the scope of the present subject matter, and additional embodiments not enumerated here also fall within the present scope. Modem lead deployment practices are increasingly varied, and the present subject matter is suited for implantation using these methods.

FIG. 7 illustrates one embodiment of a lead for implantation of an electrode into a coronary vein on the left side of the heart. In various embodiments, the lead includes a first main lead body 702, which includes a proximal portion 701 and a distal portion 720. In various embodiments, the lead is constructed out of materials such as those in the discussion associated with the illustrations of FIGS. 5-6.

The lead includes, in various embodiments, lumen extending through the main lead body 702. In various embodiments, the lumen terminates in an opening in the distal end. Various aspects of the present subject matter include, but are not limited to, a lumen which has a diameter sized to slidably receive a second lead body 706. In various embodiments, the second lead body includes an integrated second electrode 708, adapted for stimulation of the left ventricle. Also, the second lead body contains one or more lumen adapted to slidably contain varied apparatus such as a guidewire.

In various embodiments, the main lead body 702 includes a lumen adapted to slidably receive a guidewire in addition to a second lead body. However, various embodiments of the present subject matter benefit from a second lead body 706 which can act as a guidewire. In various embodiments, the second lead body 706 is a polymeric material stiffened with a reinforcing material. In one example, the second lead body 706 is a cylindrical elongate portion of silicone rubber stiffened with a coiled metallic structure. In some embodiments, the second lead body 706 is stiffened in axial compression and not bending, but other embodiments are within the scope of the present subject matter. Various embodiments of the second lead body 706 also include a mechanical bias.

In various embodiments, the second lead body includes a lumen adapted to slidably receive a guidewire. During insertion of the lead into a patient, the guidewire combined with the second lead body 706 provides stiffness adequate to manipulate mechanical bias incorporated into the design of reinforcement material for the main lead body 702. For example, by first inserting a guidewire into the second lead body 706, and then inserting the combined guidewire and second lead body into the main lead body 702, one can use a curve shaped mechanical bias located at the distal portion 720 of the main lead body 702 to tune the approach angle of the distal portion as it is moved through vasculature.

Once the first electrode 704 is positioned in a satisfactory location for transvascular stimulation of epicardial ganglia in the IVC-LA fat pad, the second lead body 706 is extended into cardiac vasculature, and positioned for stimulation of a left ventricle. The approach angle of the second lead body 706 is adjusted with the addition or removal of a guidewire to allow or reduce various degrees of a mechanical bias located proximal the tip of the second lead body 706. After the positioning of the second lead body 706, the guidewire is either extended beyond the tip of the second lead body, for use as a stimulation element, as a physiological information sensor, or as a physiological information transceiver. However, in additional embodiments, the guidewire is retracted. In some embodiments, the second lead body 706 does not include an opening in a distal portion through which a guidewire can extend.

Various methods are useful to indefinitely fix the main lead body 702 to the second lead body 706. In some embodiments, the main lead body 702 and the second lead body 706 are formed into a terminal at the proximal portion of the main lead assembly.

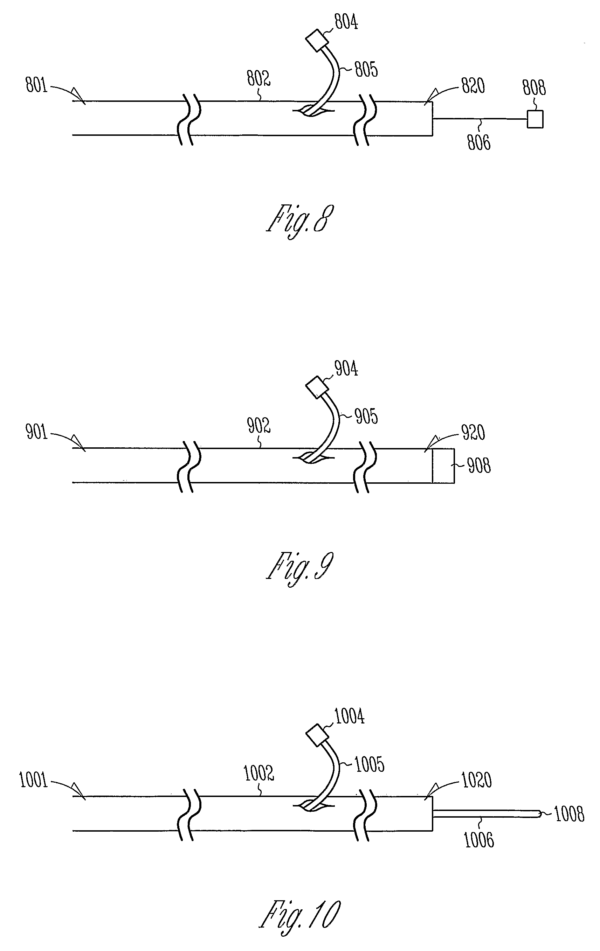

Due to the proximity of epicardial ganglia to the coronary sinus, the leads illustrated in FIGS. 8-10 include electrodes adapted for puncturing vasculature and entering the IVC-LA fat pad. The electrodes are adapted for stimulation of parasympathetic ganglia located in the IVC-LA fat pad. Various embodiments of the present subject matter deliver a neural stimulation at or near the IVC-LA fat pad in addition to the cardiac resynchronization therapy in a ventricle, using a common lead. The following examples include embodiments within this scope. It should be noted, however, that these lead illustrations should not be read as limiting other aspects and embodiments of the present subject matter.

FIG. 8 illustrates a lead adapted for implantation and delivery of neural stimulation and cardiac resynchronization therapy, according to one embodiment of the present subject matter. In various embodiments, at least two lumen extend through the main lead body from approximately the proximal portion 801 to approximately the distal portion 820.

In one embodiment, a first lumen extends to the distal portion and terminates in an opening at the distal end. In various embodiments, the lead includes a stimulation element 806 slidably placed through the first lumen in the main lead body 802. In various embodiments, the stimulation element runs from approximately the proximal portion 801 of the main lead body 802 to approximately the distal portion 820 of the main lead body 802, and in one embodiment, from approximately the proximal portion of the main lead body 802 to an opening in the distal portion, and through the opening. In various embodiments, the stimulation element 806 is integrated with a second electrode 808, including aspects discussed in the teachings of FIG. 5, however aspects of the present subject matter which are not discussed in the teachings associated with that figure additionally fall within the scope of the present subject matter. The stimulation element 806, in various embodiments, is positioned in a venous branch draining into the coronary sinus, and is adapted to stimulate the left ventricle.

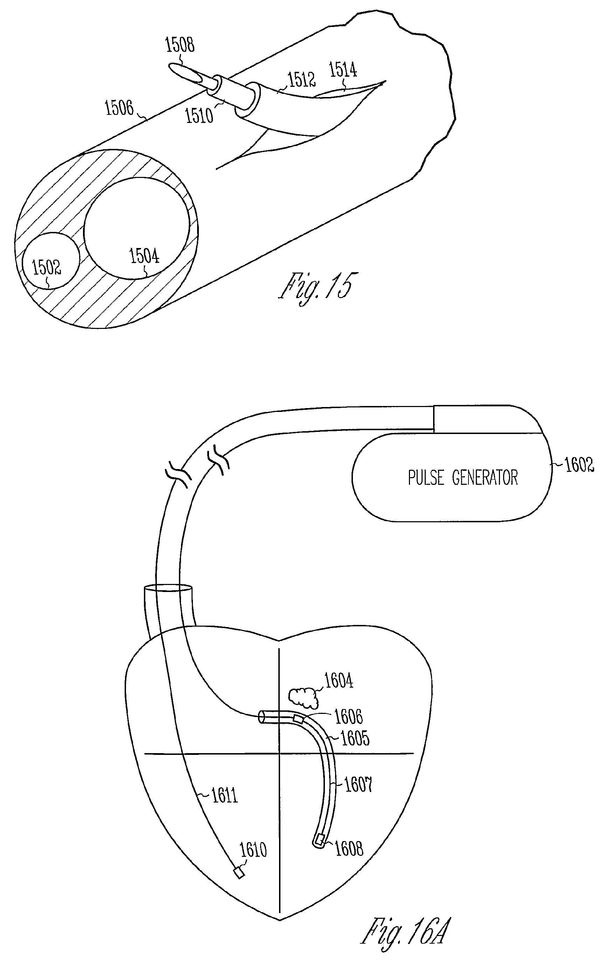

Additionally, in various embodiments, the main lead body 802 includes a puncture body lumen adapted to receive a steerable extravascular lead body 805. In various embodiments, the steerable extravascular lead body 805 includes an electrode 804. In various embodiments, the steerable extravascular lead body 805 and electrode 804 are adapted to puncture tissue, and place the electrode 804 into electrical communication with autonomic ganglia at or near the IVC-LA fat pad. Various aspects of a steerable extravascular lead body 805 and the electrode 804, are discussed in the teachings associated with FIG. 15, however, aspects of the present subject matter which are not discussed in the teachings associated with that figure additionally fall within the scope of the present subject matter.

FIG. 9 illustrates a lead adapted for implantation and delivery of neural stimulation and cardiac resynchronization therapy, according to one embodiment of the present subject matter. In various embodiments, the lead includes a main lead body 902, with a proximal end 901 and a distal end 920. In one embodiment, the main lead body 902 is integrated with a second electrode 908, including structural aspects discussed in the teachings of FIG. 6, however aspects of the present subject matter which are not discussed in the teachings associated with that figure additionally fall within the scope of the present subject matter.

Additionally, in various embodiments, the main lead body 902 includes features adapted for positioning an electrode at or near the IVC-LA fat pad. For example, in one embodiment, the main lead body 902 includes a lumen adapted to receive a steerable extravascular lead body 905. In various embodiments, the steerable extravascular lead body 905 includes an electrode 904. In various embodiments, the steerable extravascular lead body 905 and electrode 904 are adapted to puncture tissue. Various aspects of a steerable extravascular lead body 905 and an electrode 904, the combination adapted for puncturing tissue, are discussed in the teachings associated with FIG. 15; however aspects of the present subject matter which are not discussed in the teachings associated with that figure additionally fall within the scope of the present subject matter.

FIG. 10 illustrates a lead adapted for implantation and delivery of neural stimulation and cardiac resynchronization therapy, according to one embodiment of the present subject matter. In various embodiments, at least two lumen extend through the main lead body from approximately the proximal portion 1001 to approximately the distal portion 1020.

In one embodiment, a first lumen extends to the distal portion and terminates in an opening at the distal end. In various embodiments, the lead includes a second lead body 1006 slidably disposed in the first lumen of the main lead body 1002. For example, the second lead body is composed of a polymer stiffened by a reinforcing material, for example, silicone rubber containing a coiled metallic element. Additionally, the second lead body 1006 includes a third lumen, which, in various embodiments, is adapted to slidably receive a guidewire.

In various embodiments, the second lead body 1006 runs from approximately the proximal portion 1001 of the main lead body 1002, to approximately the distal portion 1020, and in one embodiment, from approximately the proximal portion 1001 of the main lead body 1002 to an opening in the distal end of the main lead body 1002, and through the opening. In various embodiments, the second lead body 1006 is integrated with a second electrode 1008, including aspects discussed in the teachings of FIG. 7, however aspects of the present subject matter which are not discussed in the teachings associated with that figure additionally fall within the scope of the present subject matter. The second lead body 1006, in various embodiments, is positioned in a cardiac vein draining into the coronary sinus, and is adapted to position the second electrode 1008 for stimulation of the left ventricle.

Additionally, in various embodiments, the main lead body 1002 includes a lumen adapted to receive a steerable extravascular lead body 1005. In various embodiments, the steerable extravascular lead body 1005 includes an electrode 1004. In various embodiments, the steerable extravascular lead body 1005 and electrode 1004 are adapted to puncture tissue, and are additionally adapted to place the electrode 1004 into electrical communication with autonomic ganglia in the IVC-LA fat pad. Various aspects of a steerable extravascular lead body 1005 and the electrode 1004 are discussed in the teachings associated with FIG. 15, however aspects of the present subject matter which are not discussed in the teachings associated with that figure additionally fall within the scope of the present subject matter.

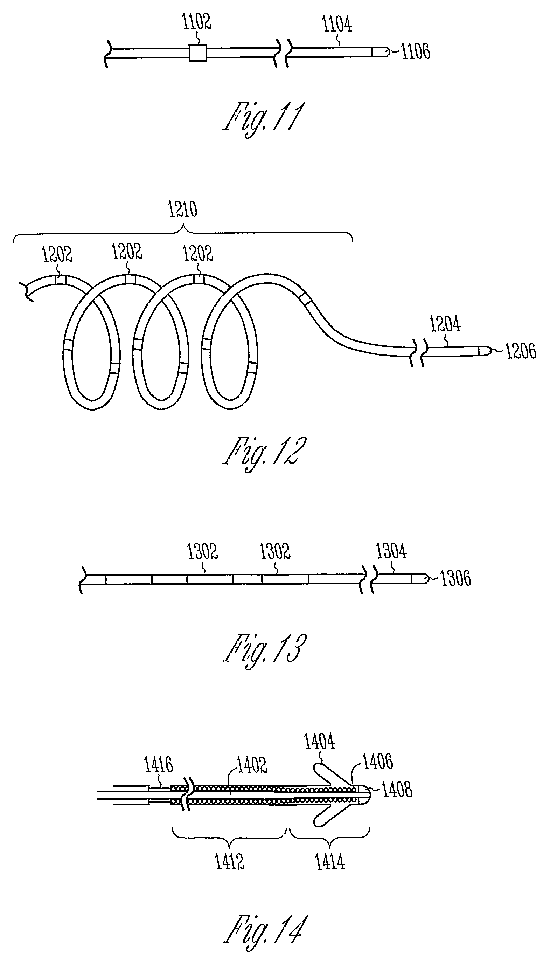

FIG. 11 shows a lead assembly adapted to deliver neural stimulation with a ring electrode, and ventricular stimulation with a tip electrode, according to various embodiments of the present subject matter. Various embodiments of the lead illustrated include aspects of the lead discussed in FIG. 6, however aspects of the present subject matter which are not discussed in the teachings associated with that figure additionally fall within the scope of the present subject matter.

In various embodiments, the lead includes a main lead body 1104 constructed from a biocompatible material, and including a rigidity adapted for placement in vasculature. In various embodiments, the lead includes a ring electrode 1102. In various embodiments, the ring electrode structure includes at least one conductive band at least partially circumscribing the main lead body. In various embodiments, the ring electrode includes one or more regions placed in electrical communication with one or more conductors running through the main lead body 1104 to a terminal suitable for connection to an implantable cardiac rhythm management device.

In various embodiments, the ring electrode 1102 includes an exposed metallic surface connected to the metallic coil which runs the length of the main lead body 1104. Additionally, the lead includes a tip 1106 adapted for providing an electrical pulse to a patient. In particular, the main lead body 1104 is sized to place the electrodes 1102 proximal the IVC-LA fat pad, and, additionally, to place electrode 1106 proximal the left ventricle, typically in a cardiac vein, the electrode 1106 positioned to deliver ventricular stimulation.

FIG. 12 shows a series of ring electrodes adapted to deliver neural stimulation combined with a lead with a mechanical bias, and a tip electrode adapted to deliver ventricular stimulation, according to one embodiment of the present subject matter. The mechanical bias illustrated is helical in shape, but other shapes are within the scope of the present subject matter. Various embodiments of the lead illustrated include aspects of the lead discussed in FIG. 6, however aspects of the present subject matter which are not discussed in the teachings associated with that figure additionally fall within the scope of the present subject matter.

In various embodiments, the lead includes a mechanical bias 1210 resulting in a helix shape. The bias is relieved by the insertion of a guidewire, in various embodiments of the present subject matter. The bias is useful for positioning the lead in vasculature. For example, in one embodiment, the outer diameter of the helix shape is sized to mate to the inner diameter of the coronary sinus proximal the IVC-LA fat pad, and the bias aids in putting one or more electrodes 1202 in electrically communication with the patient vasculature.

One feature present in some embodiments is an array of electrodes. In various embodiments, the electrodes include individually wired elements capable of individually delivering an electronic pulse. In various embodiments, each individual element is in electrical communication with an individual terminal, typically positioned near a proximal portion, and suited for connection to an implantable pulse generator. By using an array of electrodes, rather than a single electrode, transvascular stimulation is more effective. For example, a physician can tune the bias 1210 to deliver an improved form of neural stimulation by adjusting the energy delivered to several regions of the electrodes 1202.

Additionally, the lead includes a tip 1206 adapted for providing an electrical pulse to a patient. In particular, the main lead body 1204 is sized to place the electrodes 1202 proximal the IVC-LA fat pad, and, additionally, to place electrode 1206 proximal the left ventricle, such as in a cardiac vein. The electrode 1206 positioned to deliver ventricular stimulation.

Helical electrodes within the scope of the present subject matter include those taught in the following commonly assigned U.S. Patent is related and incorporated herein by reference: "Leads for Pacing and/or Sensing the Heart from Within the Coronary Veins," U.S. Patent No. 6,584,362, filed August 30, 2000, issued June 24, 2003.

FIG. 13 shows a lead with at least one ring electrode 1302 adapted to deliver neural stimulation, and a tip electrode adapted to deliver ventricular stimulation, according to various embodiments of the present subject matter. Various embodiments include aspects of the lead discussed in FIG. 12.

The lead illustrates one example of a lead with one or more electrode sites 1302 for stimulation of parasympathetic ganglia in the IVC-LA fat pad. Additionally, the example illustrates electrode sites 1302 integrated with a main lead body 1304, and also illustrates one embodiment of a lead without a mechanical bias.

The lead additionally includes a tip 1306 adapted for providing an electrical pulse to a patient. In particular, the main lead body 1304 is sized to place the electrodes 1302 proximal the IVC-LA fat pad, and, additionally, to place electrode 1306 proximal the left ventricle, typically in a cardiac vein, the electrode 1306 positioned to deliver ventricular stimulation.

FIG. 14 shows a partial cross section of a lead with at least one ring electrode adapted to deliver neural stimulation, and a tip electrode adapted to deliver ventricular stimulation, according to various embodiments of the present subject matter. Various embodiments of the illustrated lead include aspects of the lead discussed in FIG. 6, however aspects of the present subject matter which are not discussed in the teachings associated with that figure additionally fall within the scope of the present subject matter.

The lead includes a ring electrode 1416, adapted for providing electrical stimulation to parasympathetic ganglia located in the IVC-LA fat pad. Additionally, the lead includes a tip suitable for implantation proximal the left ventricle. In particular, the lead includes a reinforcement element 1406 adapted to be deformed to be implanted in a cardiac vein. The lead can include a mechanical bias, and various embodiments include a curve which assists in placement of the lead in vasculature.

For example, in one embodiment, the lead includes a guidewire 1402 of various diameters, including a thicker portion 1412, a thinner portion 1414, and a taper disposed between the two portions. In various embodiments, by positioning the guidewire in different places relevant to the reinforcing element 1406, one can manipulate the lead for vascular implantation.

In various embodiments, the lead additionally includes tines 1404 and a tip 1408 which are adapted for fostering tissue growth, the new tissue serving to anchor the lead into position.

FIG. 15 shows a perspective view including a cross section of a lead adapted to deliver neural stimulation using at least one electrode for puncturing vasculature, according to one embodiment of the present subject matter.

In various embodiments, the lead includes a main lead body 1506. The main lead body 1506 includes, in various embodiments, a puncture body lumen 1504 adapted for passage of a steerable extravascular lead body, and a second lumen 1502 adapted for passage of a guidewire, stylet or cardiac resynchronization therapy lead. In various embodiments, the main lead body 1506 is made from a resilient material, such as silicon rubber, which, in some examples, has a rigid reinforcing material.

The steerable extravascular lead body 1512, in various embodiments, includes several aspects useful for puncturing tissue, and in particular, vasculature. In one embodiment, the steerable extravascular lead body includes an elongate cylindrical portion which is cut at an angle, providing a sharpened edge adapted to puncture tissue.

The steerable extravascular lead body additionally includes aspects which are helpful for placing an implantable pulse generator connected to a lead into electrical communication with autonomic ganglia, and in particular, ganglia present in the IVC-LA fat pad. For example, in some embodiments, the steerable extravascular lead body 1512 is disposed in the puncture body lumen 1504. The steerable extravascular lead body 1512, in various embodiments, is a polymeric material which either is sufficiently stiff to enable the steerable extravascular lead body 1512 to burst through the walls defining the puncture body lumen 1504 to the exterior of the main lead body 1506, or is reinforced. For example, in one embodiment, the steerable extravascular lead body 1512 includes a reinforcing material including a mechanical bias, which forces the steerable extravascular lead body 1512 into a curve, and out of axial alignment with the puncture body lumen 1504. In one embodiment, the strength of the mechanical bias is selected to enable the steerable extravascular lead body 1512 to burst through the wall of the puncture body lumen 1504. Additionally, the mechanical bias is manipulated so as to enable straightening when combined with a guidewire or other element suitable for guiding the main body through vasculature. Alternatively, the lead body 1506 could be manipulated using a bias and guidewire in lumen 1502 to create a corner around which extravascular lead 1512 would not follow, but exit lumen 1504.

For example, in one embodiment, the extravascular lead body 1512 includes a lumen. Straightening the extravascular lead body 1512, in various embodiments, is accomplished by inserting one or more components into the lumen. In one embodiment, a probe 1508 is inserted. The probe, in various embodiments, is a conductive member adapted for electrical communication with parasympathetic ganglia located in the IVC-LA fat pad. The probe 1508 can include a tapered tip with a sharp apex, adapted to puncture tissue. The probe, as such, is useful for puncturing tissue after the steerable extravascular lead body 1512 has punctured the puncture body lumen 1504.

Phrased otherwise, in one embodiment, the steerable extravascular lead body 1512 includes a bias forming a curvature. During implantation, a probe 1508 is inserted to straighten the bias. The steerable extravascular lead body 1512 is then positioned in a first position, disposed in the main lead body 1506 in a location which is desirable for puncturing tissue and placing an electrode in electrical communication with parasympathetic ganglia located in the IVC-LA fat pad. In a second position, the probe is removed, restoring the bias in steerable extravascular lead body 1512, the bias being of a sufficient strength to puncture the wall of the puncture body lumen 1504 in the main lead body 1506, the probe 1508 then being reinserted and put into electrical communication with parasympathetic ganglia of the IVC-LA fat pad.

Positioning the probe, however, is further improved by using a second body 1510, which, in various embodiment, can include a secondary bias. In various embodiments, once the steerable extravascular lead body 1512 punctures the walls of the puncture body lumen 1504, reinsertion of elements internal to the steerable extravascular lead body 1512 tends to introduce forces which urge the steerable extravascular lead body 1512 back into coaxial alignment with the puncture body lumen 1504. One way to reduce the effect of this tendency is to use probe 1508 and a secondary body 1510. For example, in one embodiment, a guidewire of a high stiffness is used to initially extend the main body into position, and then to release the main body bias. In various embodiments, the main body punctures the puncture body lumen 1504 of the main lead body 1506. The lumen through which the guidewire passed is filled with the combined probe 1508 and secondary body 1510, in various embodiments. In one example, the combined probe and secondary body is less stiff than the guidewire.

In various embodiments, the secondary body includes a bias, and the probe serves to eliminate the bias during implantation. By changing the location of the probe within the secondary body, various degrees of approach angle to tissue is achieved, resulting in a secondary body which is directed through tissue. In some examples, combining the bias of the main body and the secondary body enables one to obtain improved maneuverability with respect to the final positioning of the probe 1508.

In various embodiments, the lead includes aspects which promote vascular healing after puncture. For example, in one embodiment, a portion of the steerable extravascular lead body includes extracellular matrix (ECM). ECM includes decellularized xenogeneic or allogeneic isolated ECM, e.g., ECM isolated from small intestinal submucosa. The term "isolated" when used in relation to ECM, refers to a complex of molecules that is identified and/or separated from at least one contaminant biological component with which it is ordinarily associated in its natural source. Isolated ECM is present in a form or setting that is different from that in which it is found in nature.

Isolated ECM is a unique biomaterial with unique properties, e.g., isolated ECM is biocompatible, e.g., has low immunogenicity, biodegradable, anti-thrombotic, anti-inflammatory and/or anti-bacterial, and optionally has mechanical and regenerative properties, e.g., the modulation of fibrosis, promotion of cell infiltration, or promotion of deposition of host derived neomatrix. Thus, when ECM is used with an implanted device, the performance of that device may be improved. For example, when ECM is used as an external interface layer between a patient and an implanted device such as a lead, the chronic performance and patient tolerance of these devices may be improved.

ECM may be isolated from endothelial layers of various cell populations, tissues and/or organs. In one embodiment, ECM is isolated from any organ or tissue source including the dermis of the skin, liver, alimentary, respiratory, intestinal, urinary or genital tracks of a warm blooded vertebrate. ECM employed in the invention may be from a combination of sources. Isolated ECM may be prepared as a sheet, in particulate form, gel form and the like.

In one embodiment, ECM is isolated from the small intestine. Intestinal submucosal tissue for use in the invention typically comprises the tunica submucosa delaminated from both the tunica muscularis and at least the luminal portions of the tunica mucosa. In one embodiment, the submucosal tissue comprises the tunica submucosa and basilar portions of the tunica mucosa including the lamina muscularis mucosa and the stratum compactum. The preparation of submucosal tissue is described in U.S. Patent No. 4,902,508 and Bell, In: Tissue Engineering: Current Perspectives, Cambridge, MA, Burkhauser Publishers, pp. 179-189 (1993). For example, a segment of vertebrate intestine, preferably harvested from porcine, ovine or bovine species, or other warm blooded vertebrates, is rinsed free of contents, then split longitudinally to form a sheet and delaminated. In particular, the superficial layers of the tunica mucosa are removed by mechanical delamination. The tissue is then turned to the opposite side and the tunica muscularis externa and tunica serosa are mechanically removed leaving the tunica submucosa and the basilar layers of the tunica mucosa. The remaining tissue represents isolated ECM and may include a small number of intact cells.

In one embodiment, ECM is isolated from the urinary bladder. The wall of the urinary bladder is composed of the following layers: the mucosa (including a transitional epithelium layer and the tunica propria), a submucosa layer, up to three layers of muscle and the adventitia (a loose connective tissue layer)--listed in crossection from luminal to abluminal sides. Urinary bladder submucosa may be prepared from bladder tissue harvested from animals raised for meat production, including, for example, porcine, ovine or bovine species or other warm-blooded vertebrates. For example, the urinary bladder is harvested and thoroughly rinsed in tap water to remove its contents. The bladder is split open through the apex and bisected to yield roughly equal-sized halves that are prepared separately. The luminal side of the bladder is placed face down and the external muscle layers, i.e., muscularis externa (smooth muscle cell layers and serosa), are removed by mechanical delamination. The transitional epithelium of the urinary bladder is removed by either mechanical or ionic methods (e.g., 1.0 N NaCl treatment) leaving behind tissue corresponding to isolated ECM, e.g., approximately a 50 *µ*M to 80 *µ*M thick sheet of ECM that originally resides between the transitional epithelium and the smooth muscle layers of the urinary bladder, i.e., the submucosa and basement membrane of the transitional epithelium.

In another embodiment, ECM from bladder wall segments or small intestine is prepared using a modification to the technique in Meezan et al. (Life Sci., 17:1721 (1975)). The method in Meezan et al. includes placing tissue fractions in a large volume (100:1) of distilled water containing 0.1% sodium azide and magnetically stirring the mixture for 1-2 hours in order to lyse the cells and release the intracellular contents. The lysed tissue suspension is then centrifuged to yield a firm pellet, and the supernatant discarded. The pellet is suspended in 40 ml of 1M NaCl and 2000 Kunitz units of DNAase (Sigma, Deoxyribonuclease 1) are added and the suspension stirred for 1-2 hours. The mixture is again centrifuged to bring down a firm pellet and the supernatant discarded. The pellet is then suspended in 40 ml of 4% sodium deoxycholate containing 0.1 % sodium azide and stirred for 2-4 hours at room temperature. The mixture is centrifuged, the supernatant discarded, and the pellet either washed several times with water by centrifugation and resuspension, or by extensive irrigation on a 44 micron nylon sieve (Kressilk Products, Inc:, Monterey Park, California). In the modified method, the time of incubation with sodium deoxycholate and sodium azide is increased and additional washing procedures incorporated. Accordingly, first, the mucosa is scraped off mechanically. Afterwards all cell structures of the remaining tissue are eliminated chemically and enzymatically leaving the acellularized muscularis layer. This is achieved with subsequent exposure to a hypoosmolar and hyperosmolar solutions of crystalloids. In addition, a final treatment with sodium deoxycholate destroys remaining cell structures. After following washing procedures, the resulting material, which provides cross-linked fibres of the submucosa with the remaining muscularis collagen-elastin framework, is stored in PBS solution, e.g., with antibiotics at 4°C for a few months. Isolated ECM is cut, rolled, or folded.

Fluidized forms of submucosal tissue are prepared by comminuting submucosa tissue by tearing, cutting, grinding, or shearing the harvested submucosal tissue. Thus, pieces of submucosal tissue are comminuted by shearing in a high speed blender, or by grinding the submucosa in a frozen or freeze-dried state, to produce a powder that can thereafter be hydrated with water or buffered saline to form a submucosal fluid of liquid, gel or paste-like consistency.

The comminuted submucosa formulation can further be treated with an enzymatic composition to provide a homogenous solution of partially solubilized submucosa. The enzymatic composition may comprise one or more enzymes that are capable of breaking the covalent bonds of the structural components of the submucosal tissue. For example, the comminuted submucosal tissue is treated with a collagenase, glycosaminoglycanase, or a protease, such as trypsin or pepsin at an acidic pH, for a period of time sufficient to solubilize all or a portion of the submucosal tissue protein components. After treating the comminuted submucosa formulation with the enzymatic composition, the tissue is optionally filtered to provide a homogenous solution. The viscosity of fluidized submucosa for use in accordance with this disclosure is manipulated by controlling the concentration of the submucosa component and the degree of hydration. The viscosity is adjusted to a range of about 2 to about 300,000 cps at 25°C. Higher viscosity formulations, for example, gels, are prepared from the submucosa digest solutions by adjusting the pH of such solutions to about 6.0 to about 7.0.

The present disclosure also contemplates the use of powder forms of submucosal tissues. In one embodiment, a powder form of submucosal tissue is prepared by pulverizing intestinal submucosa tissue under liquid nitrogen to produce particles ranging in size from 0.01 to 1 mm2 in their largest dimension. The particulate composition is then lyophilized overnight, pulverized again and optionally sterilized to form a substantially anhydrous particulate composite. Alternatively, a powder form of submucosal tissue is formed from fluidized submucosal tissue by drying the suspensions or solutions of comminuted submucosal tissue.

Submucosal tissue may be "conditioned" to alter the viscoelastic properties of the submucosal tissue. Submucosal tissue is conditioned by stretching, chemically treating, enzymatically treating or exposing the tissue to other environmental factors. The conditioning of submucosal tissue is described in U.S. Patent No. 5,275,826. In accordance with one embodiment, vertebrate derived submucosal tissues are conditioned to a strain of no more than about 20%.

In one embodiment, the submucosal tissue is conditioned by stretching the tissue longitudinally. One method of "conditioning" the tissue by stretching involves application of a given load to the submucosa for three to five cycles. Each cycle consists of applying a load to the tissue for five seconds, followed by a ten second relaxation phase. Three to five cycles produces a stretch-conditioned material. For example, submucosal tissue is conditioned by suspending a weight from the tissue, for a period of time sufficient to allow about 10 to 20% or more elongation of the tissue segment. Optionally, the material is preconditioned by stretching in the lateral dimension.

In one embodiment the submucosal tissue is stretched using 50% of the predicted ultimate load. The "ultimate load" is the maximum load that is applied to the submucosal tissue without resulting in failure of the tissue (i.e., the break point of the tissue). Ultimate load is predicted for a given strip of submucosal tissue based on the source and thickness of the material. Accordingly, one method of "conditioning" the tissue by stretching involves application of 50% of the predicted ultimate load to the submucosa for three to ten cycles. Each cycle consists of applying a load to the material for five seconds, followed by a ten second relaxation phase. The resulting conditioned submucosal tissue has a strain of less than 30%, more typically a strain from about 20% to about 28%. In one embodiment, conditioned the submucosal tissue has a strain of no more than 20%. The term strain as used herein refers to the maximum amount of tissue elongation before failure of the tissue, when the tissue is stretched under an applied load. Strain is expressed as a percentage of the length of the tissue before loading.

Typically the conditioned submucosal tissue is immobilized by clamping, suturing, stapling, gluing (or other tissue immobilizing techniques) the tissue to the support, wherein the tissue is held at its preconditioned length in at least one dimension. In one embodiment, delaminated submucosa is conditioned to have a width and length longer than the original delaminated tissue and the conditioned length and width of the tissue is maintained by immobilizing the submucosa on a support. The support-held conditioned submucosal tissue is sterilized before or after being packaged.

Preferably, isolated ECM is decellularized, and optionally sterilized, prior to storage and/or use. In one embodiment, isolated ECM has a thickness of about 50 to 250 micrometers, e.g., 100 to 200 micrometers, and is > 98% acellular. Numerous methods maybe used to decellularize isolated ECM (see, for example, Courtman et al., J. Biomed. Materi. Res., 18:655 (1994); Curtil et al., Cryobiology, 34:13 (1997); Livesey et al., Workshop on Prosthetic Heart Valves, Georgia Inst. Tech. (1998); Bader et al., Eur. J. Cardiothorac. Surg., 14:279 (1998)). For instance, treatment of isolated ECM with dilute (0.1 %) peracetic acid and rinsing with buffered saline (pH 7.0 to 7.4) and deionized water renders the material acellular with a neutral pH. Alternatively, isolated ECM is thoroughly rinsed under running water to lyse the remaining resident cells, disinfected using 0.1% peracetic acid in ethanol, and rinsed in phosphate buffered saline (PBS, pH = 7.4) and distilled water to return its pH to approximately 7.0. Decellularization may be ascertained by hematoxylin-eosin staining.

Isolated, and optionally decellularized, ECM contains a mixture of structural and functional molecules such as collagen type I, III, IV, V, VI; proteoglycans; glycoproteins; glycosaminoglycans; and growth factors in their native 3-dimensional microarchitecture, including proteins that influence cell attachment, gene expression patterns, and the differentiation of cells. Isolated ECM is optionally sterilized and may be stored in a hydrated or dehydrated state.

Isolated ECM may be sterilized using conventional sterilization techniques including tanning with glutaraldehyde, formaldehyde tanning at acidic pH, ethylene oxide treatment, propylene oxide treatment, gas plasma sterilization, gamma radiation, electric beam radiation and peracetic acid sterilization. Sterilization techniques which do not adversely affect the mechanical strength, structure, and biotropic properties of the isolated ECM are preferred. For instance, strong gamma radiation may cause loss of strength of sheets of submucosal tissue. Preferred sterilization techniques include exposing isolated ECM to peracetic acid, low dose gamma irradiation, e.g., 1-4 mRads gamma irradiation or more preferably 1-2.5 mRads of gamma irradiation, or gas plasma sterilization. In one embodiment, peracetic acid treatment is typically conducted at a pH of about 2 to about 5 in an aqueous ethanolic solution (about 2 to about 10% ethanol by volume) at a peracid concentration of about 0.03 to about 0.5% by volume. After isolated ECM is sterilized, it may be wrapped in a porous plastic wrap or foil and sterilized again, e.g., using electron beam or gamma irradiation sterilization techniques. Isolated ECM for implantation is generally subjected to two or more sterilization processes. Terminal sterilization, e.g., with 2.5 mRad (10 kGy) gamma irradiation results in a sterile, pyrogen-free biomaterial. Isolated ECM or isolated, decellularized ECM may then be stored, e.g., at 4°C, until use. Lyophilized or air dried ECM is rehydrated and used in accordance with this disclosure without significant loss of its properties. Decellularized and/or sterilized isolated ECM is substantially nonimmunogenic and has high tensile strength. Isolated ECM may, upon implantation, undergo remodeling (resorption and replacement with autogenous differentiated tissue), serve as a rapidly vascularized matrix for support and growth of new tissue, and assume the characterizing features of the tissue(s) with which it is associated at the site of implantation, which may include functional tissue.

In some embodiments, isolated ECM may be subjected to chemical and non-chemical means of cross-linking to modify the physical, mechanical or immunogenic properties of naturally derived ECM (Bellamkondra et al., J. Biomed. Mater. Res., 29:633 (1995)). Chemical cross-linking methods generally involve aldehyde or carbodiimide. Photochemical means of protein cross-linking may also be employed (Bouhadir et al., Ann. NY Acad. Sci., 842:188 (1998)). Cross-linking generally results in a relatively inert bioscaffold material which may induce a fibrous connective tissue response by the host to the scaffold material, inhibit scaffold degradation, and/or inhibit cellular infiltration into the scaffold. ECM scaffolds that are not cross-linked tend to be rapidly resorbed in contrast nonresorbable cross-linked materials or synthetic scaffolds such as Dacron or polytetrafluoroethylene (Bell, Tissue Engin., 1:163 (1995); Bell, In: Tissue Engineering: Current Perspectives, Burhauser Pub. pp. 179-189 (1993); Badylak et al., Tissue Engineering, 4:379 (1998); Gleeson et al., J. Urol., 148:1377 (1992)).

In accordance with the present disclosure isolated ECM is used advantageously to decrease undesirable sequelae at the site of device implantation in a warm blooded vertebrate. A solid sheet, strip or loop of isolated ECM, or fluidized or powder forms of isolated ECM, may be applied to and/or fixed to a device. A sheet of isolated ECM is applied to (contacted with) or adhered to (fixed to) an implantable device. Particulate isolated ECM may be coated on an implantable device, and/or a gel form of ECM may be applied to an implantable device and subsequently lyophilized to form a coating. In one embodiment, ECM in sheet form is used to form coated implantable devices. Isolated ECM may be applied to or affixed to a device or to other isolated ECM materials, other bioscaffolds or other materials with anchoring projections (such as plastic or metal pins or sutures), adhesives, or other fixation devices known to those skilled in the art. In one embodiment, an isolated ECM sheet is sutured or otherwise secured to a device. For example, isolated ECM maybe wrapped around the device and redundant tissue gathered and secured via sutures. Tissue segments or sheets are attached to each other before or during attachment to a device using surgically acceptable techniques, e.g., suturing, gluing, stapling or compressing. Multi-laminate constructs may be formed by overlapping individual strips of isolated ECM and applying pressure to the overlapped portions to fuse the strips together. In one embodiment, pressure is applied to the overlapped strips under conditions allowing dehydration of the isolated ECM.

Extracellular matrix embodiments include subject matter present in the following commonly assigned related applications : "Lead Electrode Incorporation Extracellular Matrix," Serial No. 11/017,238, filed December 20, 2004; "Implantable Medical Devices Comprising Isolated Extracellular Matrix," Serial No. 11/017,432, filed December 20, 2004; "Use of Extracellular Matrix and Electrical Therapy," Serial No. 11/017,237, filed December 20, 2004; and "Epicardial Patch Including Isolated Extracellular Matrix with Pacing Electrodes," Serial No. 11/017,627, filed December 20, 2004.

One example of an embodiment with extracellular matrix includes extracellular matrix disposed around the exterior of the secondary body 1510. In various embodiments, the steerable extravascular lead body 1512 punctures the main lead body 1506. Following puncture, an assembly capable of puncturing tissue is advanced through the main body. In one example, the assembly includes a probe 1508, and a secondary body 1510. In various embodiments, the probe 1508 and secondary body 1510 puncture vasculature, and advance until extracellular matrix coating the secondary body is in contact with the tissue opening created by the puncture. In various embodiments, the probe is additionally advanced, placing an electrode in the IVC-LA fat pad. In one example, the probe 1508 includes an electrode capable of monitoring physiological data, and additionally capable of electrically stimulating neural cells located in the IVC-LA fat pad.

In various embodiments, the lead includes a portion with a different strength than other portions of the wall of the puncture body lumen 1504, creating an area of weakened resilient material 1514. The area of weakened resilient material 1514 can assist in an effort for the steerable extravascular lead body 1512 to break through the puncture body lumen 1504 of the main lead body 1506. In various embodiments, the area of weakened resilient material is a special portion of main lead body 1506 with less material than areas near the vicinity of the area of weakened resilient material. Additionally, the area of weakened resilient material is, in various embodiments, delineated with radioopaque markers. Various radioopaque markers are within the scope of the present subject matter, including fluoroscopic materials impregnated in the lead. Radioopaque markers, in various embodiments, assist one in finding the area of weakened resilient material 1514 during fluoroscopy.

Fig. 16A shows a system diagram of a microprocessor-based cardiac device suitable for practicing the present subject matter. The device 1602 is equipped with multiple sensing and pacing channels which is configured to sense various myocardial events, and which can additionally pace multiple sites in the atria or the ventricles, or in both. For example, the device is configured for cardiac resynchronization pacing of the atria or ventricles in various embodiments of the present subject matter, and in additional embodiments, can include configurations for delivering neural stimulation intended to influence the automatic nervous system, and in particular, to induce a parasympathetic response. Additionally, the device is useful for myocardial stress reduction pacing. For example, in one embodiment, one or more cardiac sites are sensed and paced in a manner that pre-excites at least one region of the myocardium.

In one example, the system includes a first lead 1611 integrated with a first electrode 1610 adapted to stimulate the right ventricle. Additionally, the system includes a second lead 1607 integrated with a second electrode 1606 and a third electrode 1608, the second electrode 1606 adapted to transvascularly stimulate neural fibers in the IVC-LA fat pad 1604. In various embodiments, the second lead 1607 is disposed in a coronary sinus 1605. In some examples, the first electrode 1610 and the third electrode 1608 are used in conjunction to deliver cardiac resynchronization therapy.

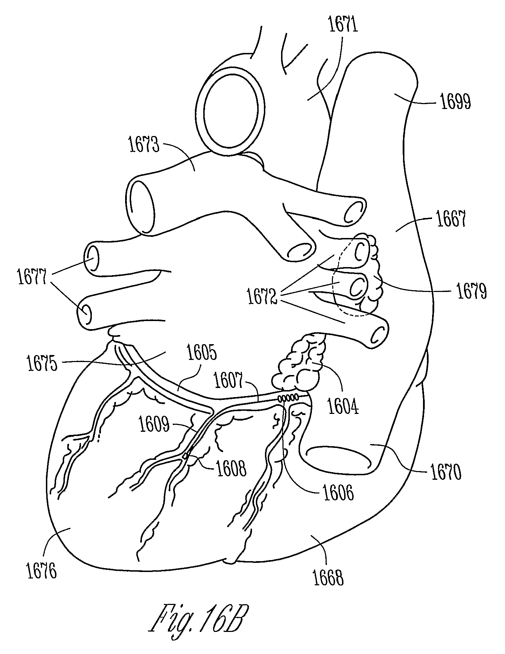

FIG. 16B illustrates the left atrium 1675, left ventricle 1676, right atrium 1667, right ventricle 1668, superior vena cava 1699, inferior vena cava 1670, aorta 1671, right pulmonary veins 1672, left pulmonary vein 1677, right pulmonary artery 1673, and coronary sinus 1605. FIG. 16B also illustrates a cardiac fat pad 1679 located proximal to the right cardiac veins and a cardiac fat pad 1604 located proximal to the inferior vena cava and left atrium.

The figures additionally illustrates an integrated lead 1607, including a third electrode 1608, and a second electrode 1606, as discussed in the teachings associated with FIG. 16A. In various embodiments, the lead is adapted for placement of the third electrode 1608 into a cardiac vein 1609.

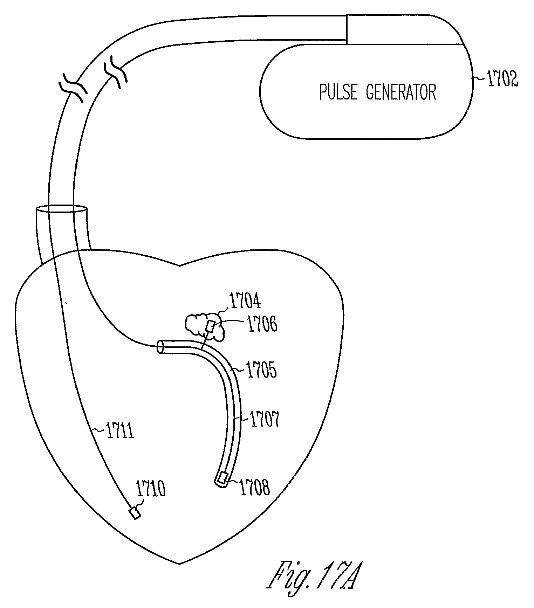

Fig. 17A shows a system diagram of a microprocessor-based cardiac device suitable for practicing the present subject matter. The device 1702 is equipped with multiple sensing and pacing channels which is physically configured to sense various myocardial events, and which can additionally pace multiple sites in the atria or the ventricles, or in both. For example, the device is configured for cardiac resynchronization pacing of the atria or ventricles in various embodiments of the present subject matter, and in additional embodiments, can include configurations for delivering neural stimulation intended to influence the automatic nervous system, and in particular, to induce a parasympathetic response. Additionally, the device is useful for myocardial stress reduction pacing. For example, in one embodiment, one or more cardiac sites are sensed and paced in a manner that pre-excites at least one region of the myocardium.

In one example, the system includes a first lead 1711 integrated with a first electrode 1710 adapted to stimulate the right ventricle. Additionally, the system includes a second lead 1707 integrated with a second electrode 1706 and a third electrode 1708, the second electrode 1706 adapted to puncture vasculature and stimulate autonomic ganglia in the IVC-LA fat pad 1704. In various embodiments, the second lead 1707 is disposed in a coronary sinus 1705. In some examples, the first electrode 1710 and the third electrode 1708 are used in conjunction to deliver cardiac resynchronization therapy.

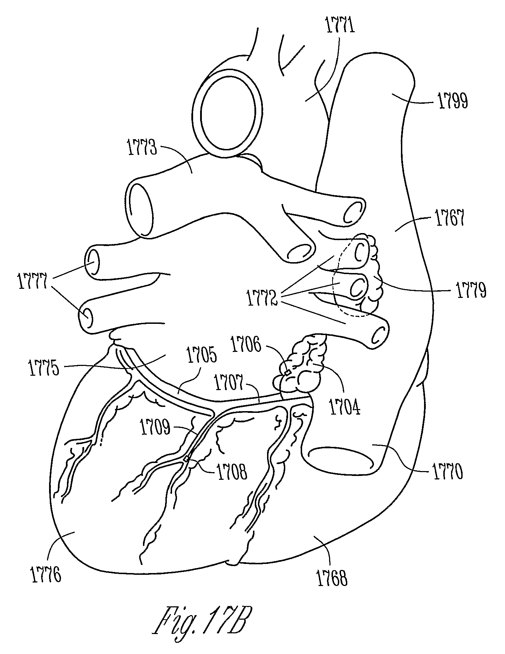

FIG. 17B illustrates the left atrium 1775, left ventricle 1776, right atrium 1767, right ventricle 1768, superior vena cava 1799, inferior vena cava 1770, aorta 1771, right pulmonary veins 1772, left pulmonary vein 1777, right pulmonary artery 1773, and coronary sinus 1705. FIG. 17B also illustrates a cardiac fat pad 1779 located proximal to the right cardiac veins and a cardiac fat pad 1704 located proximal to the inferior vena cava and left atrium.

The figures additionally illustrates an integrated lead 1707, including a third electrode 1708, and a second electrode 1706, as discussed in the teachings associated with FIG. 17A. An additional teaching of the figure demonstrates the placement of the third electrode 1708 into a cardiac vein 1709.

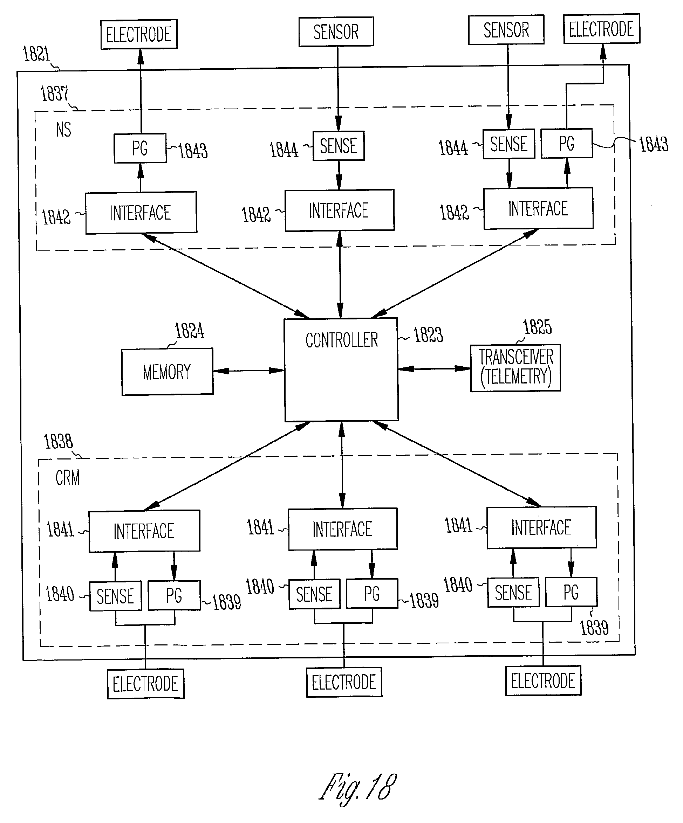

FIG. 18 illustrates an implantable medical device 1821 such as that shown at 1602 in FIG. 16A having a neural stimulation component 1837 and cardiac rhythm management component 1838, according to various embodiments of the present subject matter. The illustrated device 1821 includes a controller 1823 and a memory 1824. According to various embodiments, the controller 1823 includes hardware, software, or a combination of hardware and software to perform fat pad stimulation and cardiac rhythm management functions. For example, the programmed therapy applications discussed in this disclosure are capable of being stored as computer-readable instructions embodied in memory and executed by a processor. According to various embodiments, the controller 1823 includes a processor to execute instructions embedded in memory to perform vagal nerve stimulation and cardiac rhythm management functions, including cardiac resynchronization therapy, and functions capable of promoting reductions in myocardial stress. The illustrated device 1821 further includes a transceiver 1825 and associated circuitry for use to communicate with a programmer or another external or internal device. Various embodiments include a telemetry coil.

The cardiac rhythm management therapy section 1838 includes components, under the control of the controller, to stimulate a heart and/or sense cardiac signals using one or more electrodes. The device is equipped with multiple sensing and pacing channels which is physically configured to sense and/or pace multiple sites in the atria or the ventricles. The multiple sensing/pacing channels are configured, for example, with one atrial and two ventricular sensing/stimulation channels for delivering biventricular resynchronization therapy and neural stimulation.

The cardiac rhythm management therapy section includes a pulse generator 1839 for use to provide an electrical signal through an electrode to stimulate a heart, and further includes sense circuitry 1840 to detect and process sensed cardiac signals. An interface 1841 is generally illustrated for use to communicate between the controller 1823 and the pulse generator 1839 and sense circuitry 1840. Three electrodes are illustrated as an example for use to provide cardiac rhythm management therapy. However, the present subject matter is not limited to a particular number of electrode sites. Each electrode can include its own pulse generator and sense circuitry. However, the present subject matter is not so limited. The pulse generating and sensing functions are multiplexed to function with multiple electrodes.

The neural stimulation ("NS") therapy section 1837 includes components, under the control of the controller, to stimulate a cardiac fat pad and/or sense automatic nervous system parameters associated with nerve activity or surrogates of automatic nervous system parameters such as blood pressure and respiration. Three interfaces 1842 are illustrated for use to provide automatic nervous system therapy. However, the present subject matter is not limited to a particular number interfaces, or to any particular stimulating or sensing functions. Pulse generators 1843 are used to provide electrical pulses to an electrode for use to stimulate a cardiac fat pad. According to various embodiments, the pulse generator includes circuitry to set, and in some embodiments change, the amplitude of the stimulation pulse, the frequency of the stimulation pulse, the burst frequency of the pulse, and the morphology of the pulse such as a square wave, triangle wave, sinusoidal wave, and waves with desired harmonic components to mimic white noise or other signals.

Sense circuits 1844 are used to detect and process signals from a sensor, such as a sensor of nerve activity, blood pressure, respiration, and the like. The interfaces 1842 are generally illustrated for use to communicate between the controller 1823 and the pulse generator 1843 and sense circuitry 1844. Each interface, for example, is used to control a separate lead. Various embodiments of the neural stimulation therapy section only include a pulse generator to stimulate a cardiac fat pad. For example, the neural stimulation therapy section provides therapy promoting parasympathetic response.

One aspect of the present subject matter relates to a chronically-implanted stimulation system specially designed to minimize cardiac malfunction by stimulating parasympathetic ganglia to activate the autonomic reflex. Parasympathetic ganglia are located, for example, in a fat pad located proximal the inferior vena cava and the left atrium. In various embodiments, the system is integrated into a pacemaker / defibrillator or other electrical stimulator system. Components of the system include a high-frequency pulse generator, sensors to monitor blood pressure or other pertinent physiological parameters, leads to apply electrical stimulation to a cardiac fat pad, algorithms to determine the appropriate time to administer stimulation, and algorithms to manipulate data for display and patient management.

Various embodiments relate to a system that seeks to deliver electrically mediated neural stimulation therapy, such as therapy promoting parasympathetic response, to patients. Various embodiments combine a "stand-alone" pulse generator with a minimally invasive, unipolar lead that directly stimulates vagal nerves in the vicinity of the heart, such as in the IVC-LA fat pad. This embodiment is such that general medical practitioners lacking the skills of specialist can implant it. Various embodiments incorporate a simple implanted system that can sense parameters indicative of blood pressure. This system adjusts the therapeutic output (waveform amplitude, frequency, etc.) so as to maintain a desired quality of life. In various embodiments, an implanted system includes a pulse generating device and lead system, the stimulating electrode of which is positioned near endocardial vagal nerve tissues using transvenous implant technique(s).

Another embodiment includes a system that combines neural stimulation therapy with other therapies, such as cardiac resynchronization therapy. Some embodiments use an additional "fat pad lead" that emerges from the device header and is paced from a modified traditional pulse generating system.

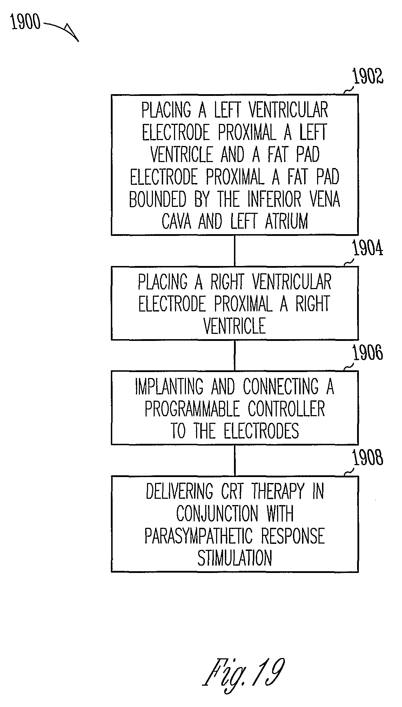

FIG. 19 shows a method 1900 for providing neural stimulation therapy and cardiac resynchronization therapy, according to one embodiment of the present subject matter. In various embodiments, placing a left ventricular electrode proximal a left ventricle and a fat pad electrode proximal a fat pad bounded by the inferior vena cava and left atrium 1902. Additionally, some examples of the method include placing a right ventricular electrode proximal a right ventricle 1904. Also, various embodiments include implanting and connecting a programmable controller to the electrodes 1906. In additional embodiments, the method includes delivering cardiac resynchronization therapy in conjunction with parasympathetic response stimulation 1908.

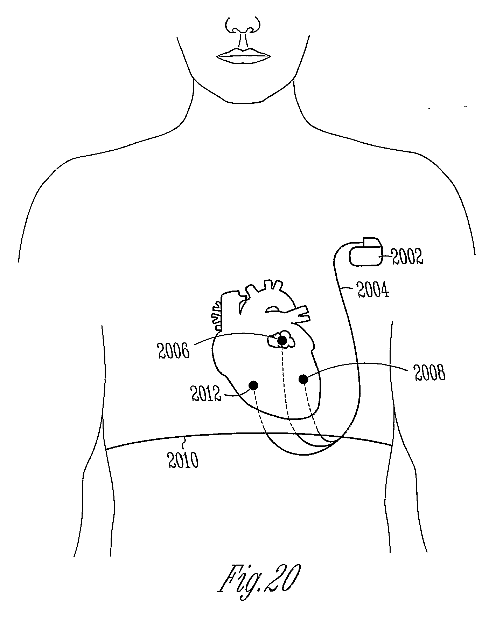

FIG. 20 shows a system diagram of a microprocessor-based cardiac device suitable for practicing the present subject matter, according to one embodiment of the present subject matter. A device 2002, connected by one or more leads 2004, interfaces with various aspects of patient anatomy to deliver parasympathetic response therapy through stimulation of parasympathetic ganglia in the IVC-LA fat pad simultaneous with delivering cardiac resynchronization therapy, using stimulation elements which are not placed in a traditional vascular manner, but which are inserted in a transxiphoidal process. For example, various embodiments place a first electrode 2012 proximal the right ventricle is implanted using a transxiphoidal process. Additional embodiments place a second electrode 2006 proximal the IVC-LA fat pad using a transxiphoidal process. One embodiment placed the second electrode 2006 partially within the IVC-LA fat pad transxiphoidal process. Also, various embodiments place a third electrode 2008 in a patient proximal the left ventricle using a transxiphoidal placement procedure.

In various embodiments, a transxiphoidal process places the electrodes in a patient by inserting them under the ribcage, and superior to the diaphragm 2010. In various embodiments, one or more electrodes are placed proximal target sites, and are either connected directly to myocardium, or are connected to epicedium, using various methods, including screw shaped electrodes, or electrodes which clasp portions of anatomy.

A transxiphoidal approach to the placement of the electrodes discussed in these teaching removes some size restrictions. For examples, a transxiphoidal lead can include one or more steering cables useful for directing a lead through tissue. Additionally, a transxiphoidal approach enables use of varied types of electrodes which normally could not be deployed through an intravenous approach.

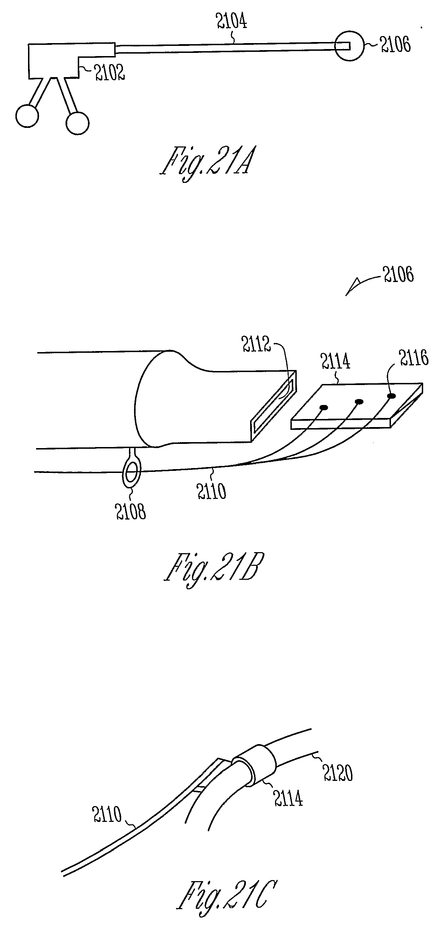

Transthoracic approaches also can be used. For example, FIGS. 21A-21B illustrate various apparatus useful for deploying one or more electrodes in a transthoracic process, according to various embodiments of the present subject matter.

FIG. 21A illustrates a tool used for deploying one or more electrodes in a transthoracic process, according to one embodiment of the present subject matter. The tool, in various embodiments, includes a graspable portion 2102 adapted to provide leverage. In various embodiments, by holding the graspable portion 2102, one can insert an elongate rod 2104 into a patient in a transthoracic fashion. In various embodiments, the tool includes a tip 2106 adapted to manipulate and deploy an electrode.

FIG. 21B illustrates on embodiment of a tip adapted to manipulate and deploy an electrode, according to one embodiment of the present subject matter. In various embodiments, the tip 2106 is adapted to carry one or more electrodes 2116, and deploy the one or more electrodes 2116 in a selective manner to various aspects of patient physiology. In various embodiments, the one or more electrodes 2116 are connected to a lead 2110 for connection to an implantable programmable pulse generator. For example, in one embodiment, one or more electrodes 2116 electrodes are deployed and fixed to the IVC-LA fat pad.

The tip 2106 and substrate 2114, in various embodiments, are adapted to position one or more electrodes 2116. In one embodiment, the tip 2106 is adapted to carry the substrate 2114 to control a mechanical bias. For example, in one embodiment, the tip 2106 includes an opening 2112 to which the substrate 2114 is mated. As the substrate 2114 is fed out of the opening 2112, the mechanical bias causes the substrate 2114 to curl, in various embodiments. As the substrate 2114 begins to curl, it affixes to patient physiology, such as the IVC-LA fat pad.

It should be noted that other electrode embodiments and electrode fixations are suitable for transthoracic deployment and are within the scope of the present subject matter.

In various embodiments, the substrate 2114 is a carrier for multiple electrodes. For example, one embodiment includes three electrodes. In this example, various electrodes 2116 are useful for adjusting device operation to improve efficiency of neural stimulation. For example, a combination of varied energy levels delivered to the electrodes 2116 improves neural capture and reduces energy requirements.

Additionally, in various embodiments, the tool is adapted to position an electrical lead 2110 in a patient. In one example, the tool includes carrying apparatus 2108 adapted for holding a lead and deploying the lead to a desired portion of patient anatomy.

FIG. 21C illustrates one example of a deployed lead 2110 and a deployed electrode substrate 2114. In various embodiments, an electrode substrate may grasp aspects of patient anatomy. One example grasps a vein 2120, however other aspects of patient physiology are compatible with the teachings of the present subject matter, and the present discussion should not be interpreted as exhaustive of exclusive.

A system according to these embodiments is used to augment partially successful treatment strategies. As an example, undesired side effects can limit the use of some pharmaceutical agents. The combination of a system according to these embodiments with reduced drug doses is particularly beneficial.

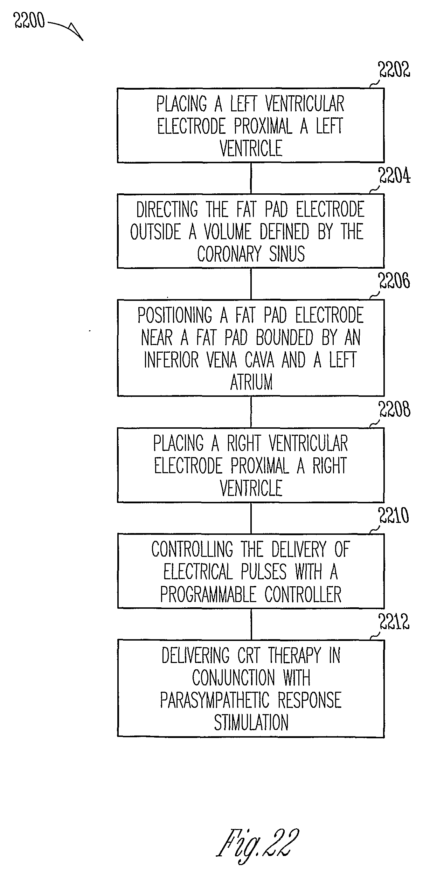

FIG. 22 shows a method 2200 for providing neural stimulation therapy and cardiac resynchronization therapy, according to one embodiment of the present subject matter. In various embodiments, the method includes placing a left ventricular electrode proximal a left ventricle 2202. Additionally the method includes directing the fat pad electrode outside a volume defined by the coronary sinus 2204. Also, the method includes positioning a fat pad electrode near a fad pad bounded by an inferior vena cava and a left atrium 2206. Additionally, some examples of the method include placing a right ventricular electrode proximal a right ventricle 2208. Also, various embodiments include controlling the delivery of electrical pulses with a programmable controller 2210. In additional embodiments, the method includes delivering cardiac resynchronization therapy in conjunction with parasympathetic response stimulation 2212.

Thus, according to various embodiments, the lead(s) and the electrode(s) on the leads are physically arranged with respect to the heart in a fashion that enables the electrodes to properly transmit pulses and sense signals from the heart, and with respect to vagal nerves to stimulate a parasympathetic response. As there is a number of leads and a number of electrodes per lead, the configuration is programmed to use a particular electrode or electrodes. According to various embodiments, the parasympathetic response is stimulated by stimulating autonomic ganglia located in the IVC-LA fat pads.

The present subject matter is suited to deliver one or both of selective neural stimulation and selective cardiac rhythm management functions. In some embodiments, the neural stimulation therapy provides therapy promoting parasympathetic response. These processes are performed by a processor executing computer-readable instructions embedded in memory, for example. These therapies include a number of applications, which have various processes and functions, some of which are identified and discussed in these teachings. The processes and functions of these therapies are not necessarily mutually exclusive, as some embodiments of the present subject matter include combinations of two or more of the identified processes and functions.

One of ordinary skill in the art will understand that the modules and other circuitry shown and described herein are implemented using software, hardware, and combinations of software and hardware. As such, the term module is intended to encompass software implementations, hardware implementations, and software and hardware implementations.

The methods illustrated in this disclosure are not intended to be exclusive of other methods within the scope of the present subject matter. Those of ordinary skill in the art will understand, upon reading and comprehending this disclosure, other methods are within the scope of the present subject matter. The above-identified embodiments, and portions of the illustrated embodiments, are not necessarily mutually exclusive. These embodiments, or portions thereof, are combined in various embodiments. For example, various embodiments combine two or more of the illustrated processes. Two or more sensed parameters are combined into a composite parameter used to provide a desired neural stimulation, and in some embodiments, therapy which promotes parasympathetic response.

In various embodiments, the methods provided above are implemented as a computer data signal embodied in a carrier wave or propagated signal, that represents a sequence of instructions which, when executed by a processor cause the processor to perform the respective method. In various embodiments, methods provided above are implemented as a set of instructions contained on a computer-accessible medium capable of directing a processor to perform the respective method. In various embodiments, the medium is a magnetic medium, an electronic medium, or an optical medium.

Although specific embodiments have been illustrated and described herein, it will be appreciated by those of ordinary skill in the art that any arrangement which is calculated to achieve the same purpose can be substituted for the specific embodiment shown. This application is intended to cover adaptations or variations of the present subject matter. It is to be understood that the above description is intended to be illustrative, and not restrictive. Combinations of the above embodiments as well as combinations of portions of the above embodiments in other embodiments will be apparent to those of skill in the art upon reviewing the above description.

## Claims

1. An apparatus, comprising;
a main lead assembly having a proximal portion adapted for connection to a device (1602) and a distal portion adapted for placement in a coronary sinus (1605) of a human heart, the distal portion terminating in a distal end for placement proximal a left ventricle;
a left ventricular electrode (1608) located at the distal end of the main lead assembly and adapted to placed in a coronary vein proximal to the left ventricle, ;
a right ventricular electrode (1610) located at the distal end of a second lead. the second lead sized for placement through a right atrium and in a right ventricle, and
a fat pad electrode (1606) disposed along the main lead assembly at a fixed distance from the distal end of the main lead assembly and the left ventricular electrode, wherein the main lead assembly is sized such that the distance between the fat pad electrode and the distal end of the main lead assembly including the left ventricular electrode is such that the fat pad electrode is located in the coronary sinus (1605) proximal to parasympathetic ganglia located in a fat pad (1604) bounded by an inferior vena cava and a left atrium and the left ventricular electrode is located in the coronary vein proximal the left ventricle when the main lead assembly is implanted in the coronary sinus; and
an implantable device (1602) connected to the proximal portion of the main lead assembly and to the second lead, the implantable device (1602) configured to use the left ventricular electrode (1608) and the right ventricular electrode (1610) to deliver cardiac resynchronization therapy stimulation pulses and to use the fat pad electrode (1606) to deliver a train of neural stimulation pulses in conjunction with the cardiac resynchronization therapy stimulation pulses to reduce ventricular wall stress.

2. The apparatus of claim 1, wherein the main lead assembly includes a main lead body extending from the proximal portion of the main lead assembly and toward the distal end of the main lead assembly, the main lead body defining at least two lumens, one of the at least two lumens being adapted to receive a second lead body.

3. The apparatus of any of claims 1-2, wherein the main lead assembly includes a main lead body extending from the proximal portion of the main lead assembly and toward the distal end of the main lead assembly; the main lead body defining at least two lumens, one of the at least two lumens being adapted to receive a stylet.

4. The apparatus of any of claims 1-3, wherein the main lead assembly includes a main lead body extending from the proximal portion of the main lead assembly and toward the distal end of the main lead assembly, the main lead body defining at least two lumens, one of the at least two lumens being adapted to receive a guidewire.

5. The apparatus of any of claims 1-4, wherein the main lead assembly includes:
a main lead body extending from the proximal portion of the main lead assembly and toward the distal end of the main lead assembly to a position beyond the fat pad electrode (1606), and connected to the fat pad electrode (1606), the main lead body defining a main lead body lumen extending from the proximal portion and terminating in an opening, the opening located in a position distal to the fat pad electrode (1606); and
a second lead body connected to the left ventricular electrode (1608) and having a near portion and a far portion, the second lead body disposed in the main lead body lumen with the near portion located at the proximal portion of the main lead body and the far portion extending past the opening located in a position distal to the fat pad electrode (1606).

6. The apparatus of claim 5, wherein the second lead body defines a second lead body lumen within the second lead body.

7. The apparatus of claim 6, wherein the second lead body lumen is adapted for passage of a slidable wire.

8. The apparatus of any of claims 5-7, wherein the fat pad electrode (1606) includes at least one conductive band at least partially circumscribing the main lead body.

9. The apparatus of claim 8, wherein for each conductive band an insulated conductor is disposed in the main lead body and connected to the conductive band, the insulated conductor extending to the proximal portion of the main lead body.

10. The apparatus of claim 9, wherein the main lead assembly includes a vascular wall abutting helix shaped bias proximal the fat pad electrode (1606).

11. The apparatus of claim 10, wherein the vascular wall abutting helix shaped bias includes predetermined bends in a wire coil in the main lead body.

12. The apparatus of any of claims 9-11, wherein the vascular wall abutting helix shaped bias includes predetermined bends in an insulator in the main lead body.

13. The apparatus of claim 1, wherein the main lead assembly has a proximal portion and a distal portion and defines a puncture body lumen extending along the main lead assembly, and further comprising:
a steerable extravascular lead body slidably disposed in the puncture body lumen, the steerable extravascular lead body having a near portion positioned at the proximal portion of the main lead assembly, and a far portion extending to a distance along the puncture body lumen to locate the far portion proximal a fat pad bounded by an inferior vena cava and a left atrium, the steerable extravascular lead body adapted to puncture the main lead assembly, wherein the fat pad electrode (1606) is connected to the steerable extravascular lead body at the far portion, the fat pad electrode (1606) adapted to stimulate parasympathetic ganglia located in the fat pad (1604) bounded by the inferior vena cava and the left atrium to reduce ventricular wall stress.

14. The apparatus of claim 13, wherein the main lead assembly includes a portion constructed of a resilient material, and an area of weakened resilient material, the area of weakened resilient material positioned adjacent the far portion of the steerable extravascular lead body.

15. The apparatus of claim 14, having a first position in which the far portion of the steerable extravascular lead body is disposed in the puncture body lumen, and a second position, in which the far portion of the steerable extravascular lead is disposed through a ruptured portion proximal the area of weakened resilient material.

16. The apparatus of any of claims 14-15, the area of weakened resilient material having at least one radioopaque marking feature.

17. The apparatus of any of claims 13-16, wherein the steerable extravascular lead body includes extracellular matrix.

18. The apparatus of claim 17, wherein the extracellular matrix is disposed along the steerable extravascular lead body at the far portion to position the extracellular matrix proximal punctured vasculature wall.

19. The apparatus of any of claims 13-18, wherein the fat pad electrode (1606) includes a conductive tissue piercing tip.

## Patentansprüche

1. Vorrichtung, welche aufweist:
eine Hauptleitungsanordnung mit einem proximalen Bereich, der für eine Verbindung mit einer Vorrichtung (1602) ausgestaltet ist, und einen distalen Bereich, der für eine Anordnung in einem Sinus coronarius (1605) eines menschlichen Herzens ausgestaltet ist, wobei der distale Bereich in einem distalen Ende für die Anordnung proximal eines linken Ventrikels endet;
eine linksventrikuläre Elektrode (1608), die sich an dem distalen Ende der Hauptleitungsanordnung befindet und ausgestaltet ist, in einer koronaren Vene proximal des linken Ventrikels angeordnet zu werden;
eine rechtsventrikuläre Elektrode (1610), die sich an dem distalen Ende einer zweiten Leitung befindet, wobei die zweite Leitung bemessen ist für die Anordnung durch einen rechten Vorhof und in einem rechten Ventrikel, und
eine Fettpolsterelektrode (1606), die sich entlang der Hauptleitungsanordnung in einem festen Abstand von dem distalen Ende der Hauptleitungsanordnung und der linksventrikulären Elektrode befindet, wobei die Hauptleitungsanordnung so bemessen ist, dass der Abstand zwischen der Fettpolsterelektrode und dem distalen Ende der Hauptleitungsanordnung enthaltend die linksventrikuläre Elektrode derart ist, dass die Fettpolsterelektrode sich in dem Sinus coronarius (1605) proximal von parasympathischen Ganglien befindet, die sich in einem Fettpolster (1604) befinden, das durch eine untere Hohlvene und einen linken Vorhof begrenzt ist, und die linksventrikuläre Elektrode sich in der koronaren Vene proximal des linken Ventrikels befindet, wenn die Hauptleitungsanordnung in den Sinus coronarius implantiert ist; und
eine implantierbare Vorrichtung (1602), die mit dem proximalen Bereich der Hauptleitungsanordnung und mit der zweiten Leitung verbunden ist, wobei die implantierbare Vorrichtung konfiguriert ist zum Verwenden der linksventrikulären Elektrode (1608) und der rechtsventrikulären Elektrode (1610), um Herzsynchronisationstherapie-Stimulationsimpulse zu liefern, und die Fettpolsterelektrode (1606) zu verwenden, um eine Reihe von Neuralstimulationsimpulsen in Verbindung mit den Herzresynchronisationstherapie-Stimulationsimpulsen zu liefern, um eine Beanspruchung der ventrikulären Wand zu verringern.

2. Vorrichtung nach Anspruch 1, bei der die Hauptleitungsanordnung einen Hauptleitungskörper enthält, der sich von dem proximalen Bereich der Hauptleitungsanordnung zu dem distalen Ende der Hauptleitungsanordnung erstreckt, wobei der Hauptleitungskörper zumindest zwei Lumen definiert, von denen eines der zumindest zwei Lumen ausgestaltet ist, einen zweiten Leitungskörper aufzunehmen.

3. Vorrichtung nach einem der Ansprüche 1 bis 2, bei der die Hauptleitungsanordnung einen Hauptleitungskörper enthält, der sich von dem proximalen Bereich der Hauptleitungsanordnung zu dem distalen Ende der Hauptleitungsanordnung erstreckt, wobei der Hauptleitungskörper zumindest zwei Lumen definiert, von denen eines der zumindest zwei Lumen ausgestaltet ist, ein Stilett aufzunehmen.

4. Vorrichtung nach einem der Ansprüche 1 bis 3, bei der die Hauptleitungsanordnung einen Hauptleitungskörper enthält, der sich von dem proximalen Bereich der Hauptleitungsanordnung zu dem distalen Ende der Hauptleitungsanordnung erstreckt, wobei der Hauptleitungskörper zumindest zwei Lumen definiert, von denen eines der zumindest zwei Lumen ausgestaltet ist, einen Führungsdraht aufzunehmen.

5. Vorrichtung nach einem der Ansprüche 1 bis 4, bei der die Hauptleitungsanordnung enthält:
einen Hauptleitungskörper, der sich von dem proximalen Bereich der Hauptleitungsanordnung zu dem distalen Ende der Hauptleitungsanordnung zu einer Position über die Fettpolsterelektrode (1606) hinaus erstreckt und mit der Fettpolsterelektrode (1606) verbunden ist, wobei der Hauptleitungskörper ein Hauptleitungskörperlumen definiert, das sich von dem proximalen Bereich erstreckt und in einer Öffnung endet, wobei sich die Öffnung an einer Position distal der Fettpolsterelektrode (1606) befindet; und
einen zweiten Leitungskörper, der mit der linksventrikulären Elektrode (1608) verbunden ist und einen Nahbereich und einen Fernbereich hat, wobei der zweite Leitungskörper in dem Hauptleitungskörperlumen angeordnet ist und sich der Nahbereich an dem proximalen Bereich des Hauptleitungskörpers befindet und der Fernbereich sich an der Öffnung, die sich an einer Position distal der Fettpolsterelektrode (1606) befindet, vorbei erstreckt.

6. Vorrichtung nach Anspruch 5, bei der der zweite Leitungskörper ein zweites Leitungskörperlumen innerhalb des zweiten Leitungskörpers definiert.

7. Vorrichtung nach Anspruch 6, bei der das zweite Leitungskörperlumen ausgestaltet ist für den Durchgang eines gleitbaren Drahts.

8. Vorrichtung nach einem der Ansprüche 5 bis 7, bei der die Fettpolsterelektrode (1606) zumindest ein leitendes Band enthält, das den Hauptleitungskörper zumindest teilweise begrenzt.

9. Vorrichtung nach Anspruch 8, bei der für jedes leitende Band ein isolierter Leiter in dem Hauptleitungskörper angeordnet und mit dem leitenden Band verbunden ist, wobei der isolierte Leiter sich zu dem proximalen Bereich des Hauptleitungskörpers erstreckt.

10. Vorrichtung nach Anspruch 9, bei der die Hauptleitungsanordnung eine an einer Gefäßwand anliegende, wendelförmige Vorspannung proximal der Fettpolsterelektrode (1606) enthält.

11. Vorrichtung nach Anspruch 10, bei der die an einer Gefäßwand anliegende, wendelförmige Vorspannung vorbestimmte Biegungen in einer Drahtspule des Hauptleitungskörpers enthält.

12. Vorrichtung nach einem der Ansprüche 9 bis 11, bei der die an einer Gefäßwand anliegende, wendelförmige Vorspannung vorbestimmte Biegungen in einem Isolator in dem Hauptleitungskörper enthält.

13. Vorrichtung nach Anspruch 1, bei der die Hauptleitungsanordnung einen proximalen Bereich und einen distalen Bereich hat und ein Durchstechkörperlumen definiert, das sich entlang der Hauptleitungsanordnung erstreckt, und weiterhin aufweist:
einen lenkbaren außervaskulären Leitungskörper, der gleitbar in dem Durchstechkörperlumen angeordnet ist, wobei der lenkbare außervaskuläre Leitungskörper einen Nahbereich, der an dem proximalen Bereich der Hauptleitungsanordnung positioniert ist, und einen Fernbereich, der sich zu einem Abstand entlang des Durchstechkörperlumens erstreckt, hat, um den Fernbereich proximal eines durch eine untere Hohlvene und einen linken Vorhof begrenzten Fettpolsters anzuordnen, wobei der lenkbare außervaskuläre Leitungskörper ausgestaltet ist, die Hauptleitungsanordnung zu durchstechen, wobei die Fettpolsterelektrode (1606) mit dem lenkbaren außervaskulären Leitungskörper an dem Fernbereich verbunden ist und die Fettpolsterelektrode (1606) ausgestaltet ist, die sich in dem Fettpolster (1604), das durch die untere Hohlvene und den linken Vorhof begrenzt ist, befindenden parasympathischen Ganglien zu stimulieren, um eine Beanspruchung der ventrikulären Wand zu verringern.

14. Vorrichtung nach Anspruch 13, bei der die Hauptleitungsanordnung einen aus einem elastischen Material gebildeten Bereich und eine Fläche aus geschwächtem elastischem Material enthält, wobei die Fläche aus geschwächtem elastischem Material angrenzend an den Fernbereich des lenkbaren außervaskulären Leitungskörpers positioniert ist.

15. Vorrichtung nach Anspruch 14, mit einer ersten Position, in der der Fernbereich des lenkbaren außervaskulären Leitungskörpers sich in dem Durchstechkörperlumen befindet, und einer zweiten Position, in der der Fernbereich der lenkbaren außervaskulären Leitung durch einen durchbrochenen Bereich proximal der Fläche aus geschwächtem elastischem Material angeordnet ist.

16. Vorrichtung nach einem der Ansprüche 14 bis 15, bei der die Fläche aus geschwächtem elastischem Material zumindest ein strahlenundurchlässiges Markiermerkmal hat.

17. Vorrichtung nach einem der Ansprüche 13 bis 16, bei der der lenkbare außervaskuläre Leitungskörper eine außerzellulare Matrix enthält.

18. Vorrichtung nach Anspruch 17, bei der die außerzellulare Matrix entlang des lenkbaren außervaskulären Leitungskörpers an dem Fernbereich angeordnet ist, um die außerzellulare Matrix proximal der durchstochenen Gefäßwand zu positionieren.

19. Vorrichtung nach einem der Ansprüche 13 bis 18, bei der die Fettpolsterelektrode (1606) eine leitende Gewebedurchstechspitze enthält.

## Revendications

1. Appareil, comprenant :
un assemblage de connexion principale comportant une partie proximale adaptée pour être connectée sur un dispositif (1602) et une partie distale adaptée pour être positionnée dans un sinus coronaire (1605) d'un coeur humain, la partie distale se terminant selon une extrémité distale pour un positionnement de façon proximale par rapport à un ventricule gauche ;
une électrode de ventricule gauche (1608) située au niveau de l'extrémité distale de l'assemblage de connexion principale et adaptée pour être positionnée dans une veine coronaire de façon proximale par rapport au ventricule gauche ;
une électrode de ventricule droit (1610) située au niveau de l'extrémité distale d'une seconde connexion, la seconde connexion étant dimensionnée pour être positionnée au travers d'une oreillette droite et dans un ventricule droit ; et
une électrode de coussinet adipeux (1606) disposée le long de l'assemblage de connexion principale à une distance fixe de l'extrémité distale de l'assemblage de connexion principale et de l'électrode de ventricule gauche, dans lequel l'assemblage de connexion principale est dimensionné de telle sorte que la distance entre l'électrode de coussinet adipeux et l'extrémité distale de l'assemblage de connexion principale incluant l'électrode de ventricule gauche soit telle que l'électrode de coussinet adipeux soit située dans le sinus coronaire (1605) de façon proximale par rapport à des ganglions parasympathiques situés dans un coussinet adipeux (1604) délimité par une veine cave inférieure et par une oreillette gauche et que l'électrode de ventricule gauche soit située dans la veine coronaire de façon proximale par rapport au ventricule gauche lorsque l'assemblage de connexion principale est implanté dans le sinus coronaire ; et
un dispositif implantable (1602) connecté à la partie proximale de l'assemblage de connexion principale et à la seconde connexion, le dispositif implantable (1602) étant configuré de manière à utiliser l'électrode de ventricule gauche (1608) et l'électrode de ventricule droit (1610) pour délivrer des impulsions de stimulation de thérapie de resynchronisation cardiaque et de manière à utiliser l'électrode de coussinet adipeux (1606) pour délivrer un train d'impulsions de stimulation neurale en conjonction avec les impulsions de stimulation de thérapie de resynchronisation cardiaque afin de réduire un stress de paroi ventriculaire.

2. Appareil selon la revendication 1, dans lequel l'assemblage de connexion principale inclut un corps de connexion principale s'étendant depuis la partie proximale de l'assemblage de connexion principale et en direction de l'extrémité distale de l'assemblage de connexion principale, le corps de connexion principale définissant au moins deux lumières, l'une des au moins deux lumières étant adaptée pour recevoir un second corps de connexion.

3. Appareil selon l'une quelconque des revendications 1 et 2, dans lequel l'assemblage de connexion principale inclut un corps de connexion principale s'étendant depuis la partie proximale de l'assemblage de connexion principale et en direction de l'extrémité distale de l'assemblage de connexion principale, le corps de connexion principale définissant au moins deux lumières, l'une des au moins deux lumières étant adaptée pour recevoir un stylet.

4. Appareil selon l'une quelconque des revendications 1 à 3, dans lequel l'assemblage de connexion principale inclut un corps de connexion principale s'étendant depuis la partie proximale de l'assemblage de connexion principale et en direction de l'extrémité distale de l'assemblage de connexion principale, le corps de connexion principale définissant au moins deux lumières, l'une des au moins deux lumières étant adaptée pour recevoir un fil de guidage.

5. Appareil selon l'une quelconque des revendications 1 à 4, dans lequel l'assemblage de connexion principale inclut :
un corps de connexion principale s'étendant depuis la partie proximale de l'assemblage de connexion principale et en direction de l'extrémité distale de l'assemblage de connexion principale jusqu'à une position au delà de l'électrode de coussinet adipeux (1606) et connecté à l'électrode de coussinet adipeux (1606), le corps de connexion principale définissant une lumière de corps de connexion principale s'étendant depuis la partie proximale et se terminant dans une ouverture, l'ouverture étant située en une position distale par rapport à l'électrode de coussinet adipeux (1606) ; et
un second corps de connexion connecté à l'électrode de ventricule gauche (1608) et comportant une partie proche et une partie éloignée, le second corps de connexion étant disposé dans la lumière de corps de connexion principale, la partie proche étant située au niveau de la partie proximale du corps de connexion principale et la partie éloignée s'étendant au delà de l'ouverture située en une position distale par rapport à l'électrode de coussinet adipeux (1606).

6. Appareil selon la revendication 5, dans lequel le second corps de connexion définit une seconde lumière de corps de connexion à l'intérieur du second corps de connexion.

7. Appareil selon la revendication 6, dans lequel le second corps de connexion est adapté pour le passage d'un fil coulissant.

8. Appareil selon l'une quelconque des revendications 5 à 7, dans lequel l'électrode de coussinet adipeux (1606) inclut au moins une bande conductrice circonscrivant au moins partiellement le corps de connexion principale.

9. Appareil selon la revendication 8, dans lequel, pour chaque bande conductrice, un conducteur isolé est disposé dans le corps de connexion principale et est connecté à la bande conductrice, le conducteur isolé s'étendant jusqu'à la partie proximale du corps de connexion principale.

10. Appareil selon la revendication 9, dans lequel l'assemblage de connexion principale inclut un moyen de poussée en forme d'hélice venant en butée contre la paroi vasculaire de façon proximale par rapport à l'électrode de coussinet adipeux (1606).

11. Appareil selon la revendication 10, dans lequel le moyen de poussée en forme d'hélice venant en butée contre la paroi vasculaire inclut des flexions prédéterminées selon une bobine filaire dans le corps de connexion principale.

12. Appareil selon l'une quelconque des revendications 9 à 11, dans lequel le moyen de poussée en forme d'hélice venant en butée contre la paroi vasculaire inclut des flexions prédéterminées selon un isolateur dans le corps de connexion principale.

13. Appareil selon la revendication 1, dans lequel l'assemblage de connexion principale comporte une partie proximale et une partie distale et définit une lumière de corps de ponction s'étendant le long de l'assemblage de connexion principale, et comprenant en outre :
un corps de connexion extravasculaire dirigeable disposé de façon coulissante dans la lumière de corps de ponction, le corps de connexion extravasculaire dirigeable comportant une partie proche positionnée au niveau de la partie proximale de l'assemblage de connexion principale et une partie éloignée s'étendant sur une distance le long de la lumière de corps de ponction afin de positionner la partie éloignée de façon proximale par rapport à un coussinet adipeux délimité par une veine cave inférieure et par une oreillette gauche, le corps de connexion extravasculaire dirigeable étant adapté pour faire ponctionner l'assemblage de connexion principale, dans lequel l'électrode de coussinet adipeux (1606) est connectée au corps de connexion extravasculaire dirigeable au niveau de la partie éloignée, l'électrode de coussinet adipeux (1606) étant adaptée pour stimuler des ganglions parasympathiques situés dans le coussinet adipeux (1604) délimité par la veine cave inférieure et par l'oreillette gauche afin de réduire le stress de paroi ventriculaire.

14. Appareil selon la revendication 13, dans lequel l'assemblage de connexion principale inclut une partie construite en matériau élastique et une zone en matériau élastique affaibli, la zone en matériau élastique affaibli étant positionnée de manière à être adjacente à la partie éloignée du corps de connexion extravasculaire dirigeable.

15. Appareil selon la revendication 14, présentant une première position dans laquelle le corps de connexion extravasculaire dirigeable est disposé dans la lumière de corps de ponction et une seconde position dans laquelle la partie éloignée de la connexion extravasculaire dirigeable est disposée au travers d'une partie rompue de façon proximale par rapport à la zone en matériau élastique affaibli.

16. Appareil selon l'une quelconque des revendications 14 et 15, la zone en matériau élastique affaibli comportant au moins une caractéristique de marquage radio-opaque.

17. Appareil selon l'une quelconque des revendications 13 à 16, dans lequel le corps de connexion extravasculaire dirigeable inclut une matrice extracellulaire.

18. Appareil selon la revendication 17, dans lequel la matrice extracellulaire est disposée le long du corps de connexion extravasculaire dirigeable au niveau de la partie éloignée afin de positionner la matrice extracellulaire de façon proximale par rapport à une paroi de vasculature ponctionnée.

19. Appareil selon l'une quelconque des revendications 13 à 18, dans lequel l'électrode de coussinet adipeux (1606) inclut une pointe conductrice de perçage de tissu.
